# EUROPEAN PATENT APPLICATION

(11) **EP 4 616 854 A1**
(43) Date of publication of application: **17.09.2025**
(21) Application number: 24162796.7
(22) Date of filing: 11.03.2024
(51) Int. Cl.: A61K 31/427, A61K 31/4402, A61K 31/5025, A61K 31/517, A61K 31/585, A61K 38/00, A61K 45/06, A61P 21/00

(54) **TREATMENT OF MUSCLE SARCOPENIA**

(71) Applicant: Molius Saglik Teknolojileri Anonim Sirketi, Ümraniye/Istanbul (TR)
(72) Inventor: MARDINOGLU, Adil, 412 60 Göteborg (SE); ALTAY, Havva Ozlem, 114 28 Stockholm (SE)
(74) Representative: Kransell & Wennborg KB

(57) **Abstract**

There is provided a compound or a pharmaceutically acceptable salt or prodrug thereof for use in a method of treatment of sarcopenia wherein the compound is selected from carbinoxamine, FR-18204, ML-167, withaferin-A, MG-132 and troglitazone.

## Description

### TECHNICAL FIELD

The present disclosure relates to the field of compounds for use in a method of treatment of sarcopenia.

### BACKGROUND

Sarcopenia is the functional decline of the skeletal muscle, and its global prevalence is estimated to be 5-50% in people above age 60 [1]. The patients typically need immediate treatment since the disease has significant personal, social, and financial consequences if it remains untreated. Currently, there is no approved therapy for efficient treatment of sarcopenia and the management is mainly restricted to lifestyle adaptation and physiotherapy [2].

Advances in high-throughput technologies and systems biology-based methodologies have revolutionized our understanding of complex biological systems in different clinical conditions. The integrative biological network analysis is widely used for revealing the molecular complexity by identifying the key genes and pathways associated with diseases [3]. The network structure enables researchers to explore connections between the nodes within modules for studying the complexity of the biological processes. Such an approach has been successfully applied in clinical research, and important discoveries have been made about the treatment of metabolic diseases and certain types of cancer [4-7]. Systems biology can be used to discover reliable biomarkers that can guide the early diagnosis of the patients and for identification of the novel drug targets can pave the way for the development of novel therapy.

Drug repositioning has gained attraction as a viable solution to overcome common bottlenecks in drug development. A variety of drugs, including metformin, niclosamide and several hormone therapies have been traditionally repurposed for the prevention and treatment of sarcopenia [8]. However, these compounds exhibited very little efficacy in clinical studies. Thus, further drug development is necessary in order to find viable and effective treatments of muscle sarcopenia.

### SUMMARY

The integration of systems biology approaches in drug repositioning has the potential to identify effective drugs and provide insights into the underlying mechanisms of diseases. For example, network-based approaches can be used to identify new drug targets by analysing protein-protein interaction networks and predicting the effects of drug-target interactions [9]. By combining network analysis with experimental validation, this approach has been successful in identifying new drug targets for a range of diseases, including cancer and cardiovascular [10]. Moreover, systems biology can aid in the identification of patient subgroups that may benefit from repurposing drugs, enabling more personalized treatments [11, 12].

The present inventors combined a systems biology approach with drug repositioning to elucidate the molecular mechanisms underlying sarcopenia and identified drug targets for the development of efficient treatment strategies for sarcopenia patients.

The inventors generated gene co-expression networks (GCNs) using muscle transcriptomics data of control subjects and sarcopenia patients, and identified the key genes and pathways associated with the progression and occurrence of sarcopenia. They further applied a drug repositioning approach to identify effective drug candidates based on gene perturbation profiles and network analysis. Furthermore, a molecular docking approach was used to show the interactions between gene targets and drug candidates and the efficacy of the drug candidates was validated in an *in vitro* model of sarcopenia.

Accordingly, there is provided a compound or a pharmaceutically acceptable salt or prodrug thereof for use in a method of treatment of sarcopenia wherein the compound is selected from carbinoxamine, FR-18204, ML-167, withaferin-A, MG-132 and troglitazone.

It has been shown that treating sarcopenic cells with a compound selected from the list above, is associated with increased expression levels of myogenic markers, myogenin (MyoG) and myosin heavy chain (MyH3). These compounds are thus especially well-suited for use in a pharmacological treatment of sarcopenia.

### BRIEF DESCRIPTION OF THE DRAWINGS

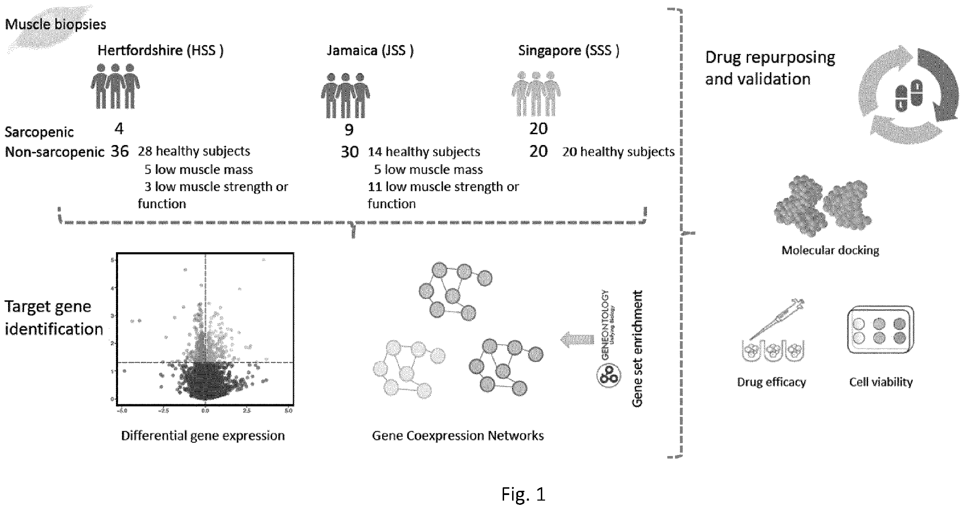

Fig 1 shows the flowchart for the study in Example 1. The transcriptomics data from the muscle samples from three sarcopenia cohorts was used in Example 1. The gene co-expression network analysis was used to identify the druggable target and then a drug repositioning approach was used to identify the effective drugs for the treatment of corresponding target genes, followed by *in vitro* validation.

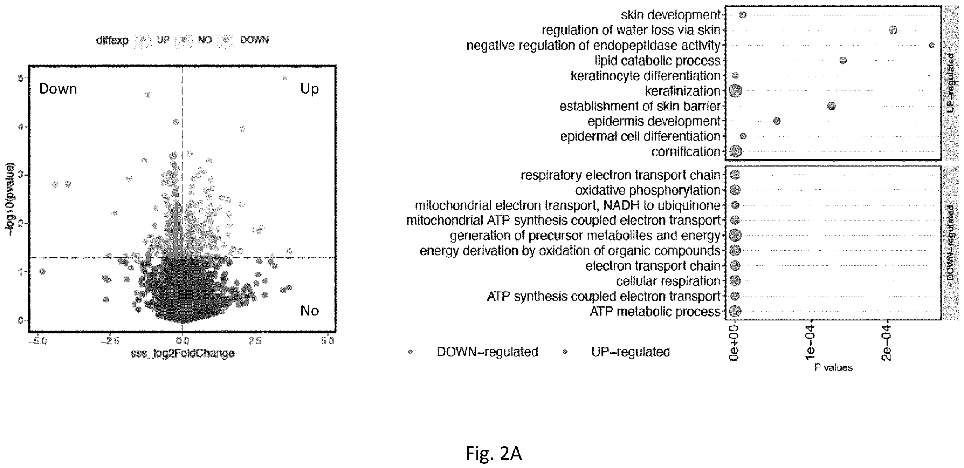

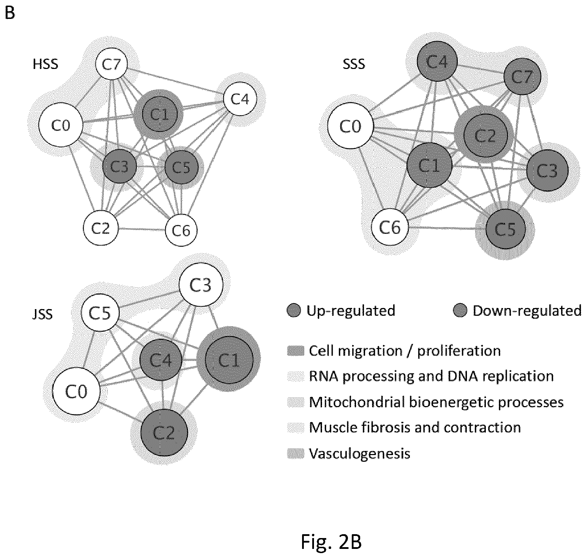

Fig 2 shows the identification of DEGs and related clusters in sarcopenia cohorts. A) Biological processes enriched in sarcopenia cohort. The x-axis represents the GO term p values B) Clusters and their corresponding biological processes in sarcopenia cohorts. The node represents one cluster in the GCN network, and the clusters significantly overlapped with upregulated DEGs were HSS-C1, SSS-C1, SSS-C2, SSS-C4, SSS-C7, JSS-C1 and JSS-C4. The clusters significantly overlapped with downregulated DEGs were HSS-C3, HSS-C5, SSS-C3, SSS-C5, JSS-C2. HSS-Co, HSS-C7, SSS-Co, SSS-C1, SSS-C6, JSS-Co, JSS-C3, JSS-C5 were enriched in RNA processing and DNA replication, HSS-C1, SSS-C2 and JSS-C1 were enriched in cell migration/proliferation, HSS-C3, SSS-C3 and JSS-C2 were enriched in mitochondrial bioenergetic processes, HSS-C4, SSS-C4, SSS-C7 and JSS-C4 were enriched in muscle fibrosis and contraction and HSS-C5 and SSS-C3 were enriched in vasculogenesis.

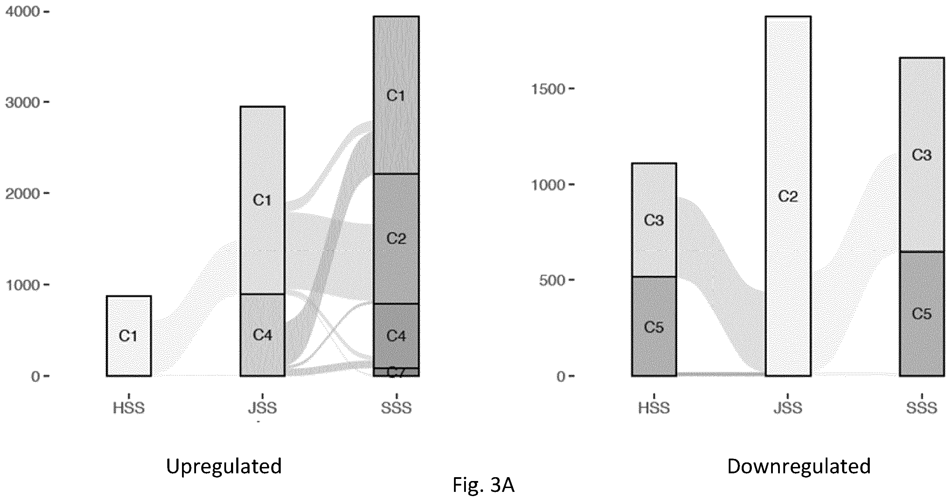

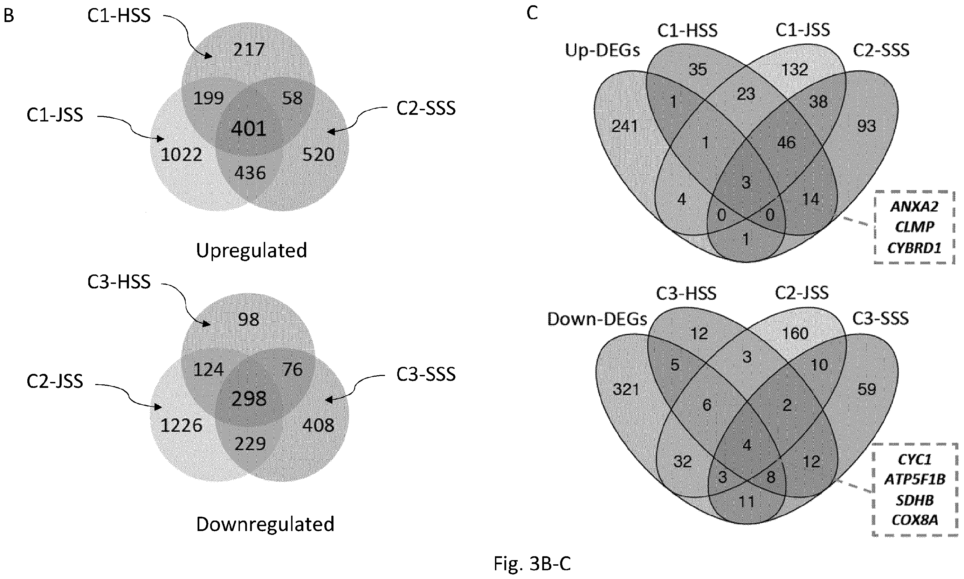

Fig 3 shows the identification of target gene. A) Upregulated and downregulated clusters in sarcopenia cohorts. The x-axis indicates the cohort names, the y-axis represents the gene number in the corresponding cluster. B) Venn diagrams of significantly overlapped clusters from the sarcopenia cohorts. C) Gene overlap between top cluster genes and DEGs with different regulate directions.

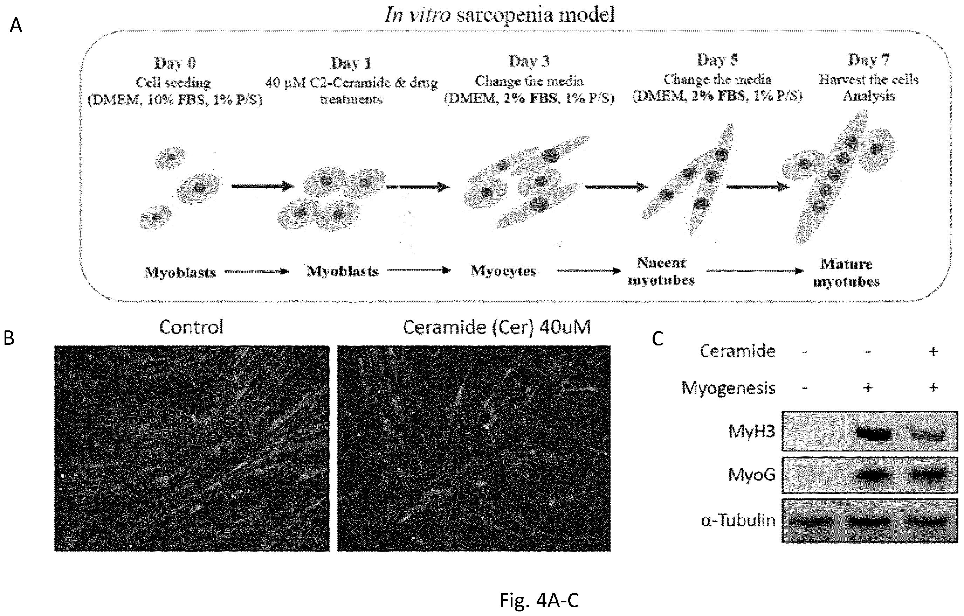

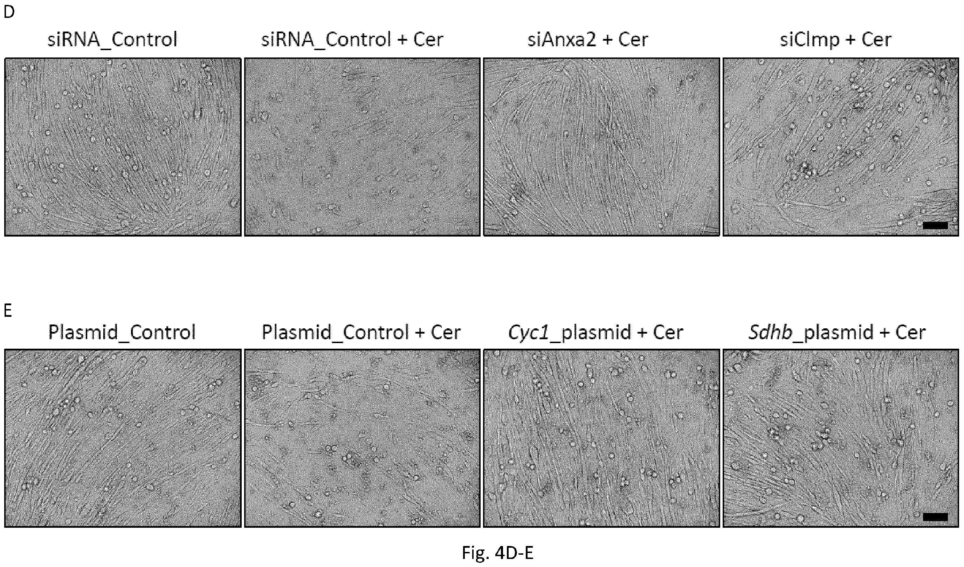

Fig 4 shows the *in vitro* model of sarcopenia with C2C12 and transfection of siRNA or Plasmid of target genes A) The diagram of *in vitro* model of sarcopenia with C2C12 cell line. B) Phenotypic change of C2C12 after 4 days of differentiation with 40 µM ceramide treatment before differentiation. Scale bar: 100 µm. C) The protein expression levels of myosin heavy chain 3 (MyH3) and myogenin (MyoG). Used α-Tubulin as housekeeping gene. D) The transfection of siRNAs with ceramide. Scale bar: 100 µm. (E) The transfection of plasmids with ceramide. Scale bar: 100 µm.

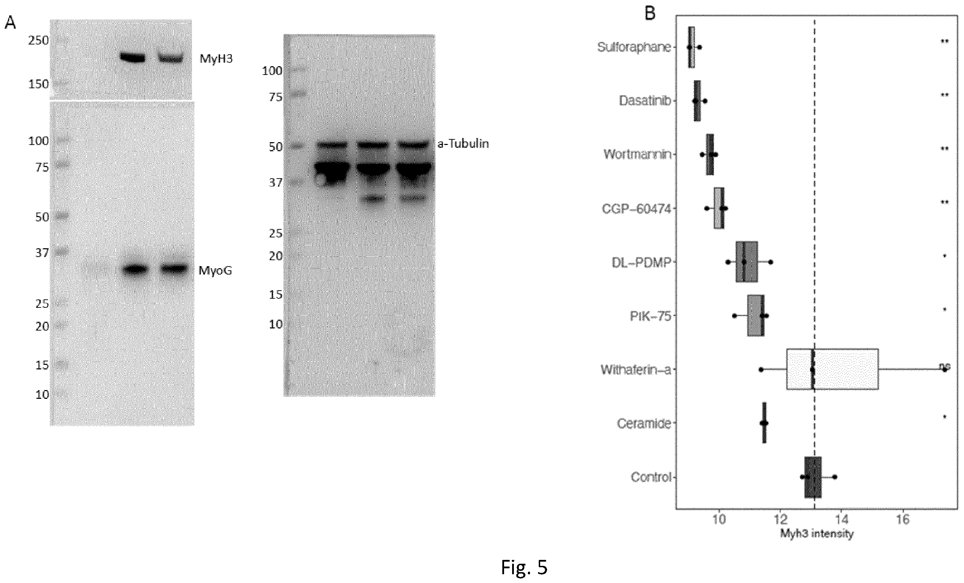

Fig 5 shows A) The uncropped western blot of protein expression levels of myosin heavy chain 3 (MyH3) and myogenin (MyoG). B) Compared to the control group, MyH3 intensity were significantly deceased in ceramide group, and showed no statistical difference in withaferin-a treated group (where ns, * and ** denote p>0.05, p<=0.05 and p<=0.01, respectively).

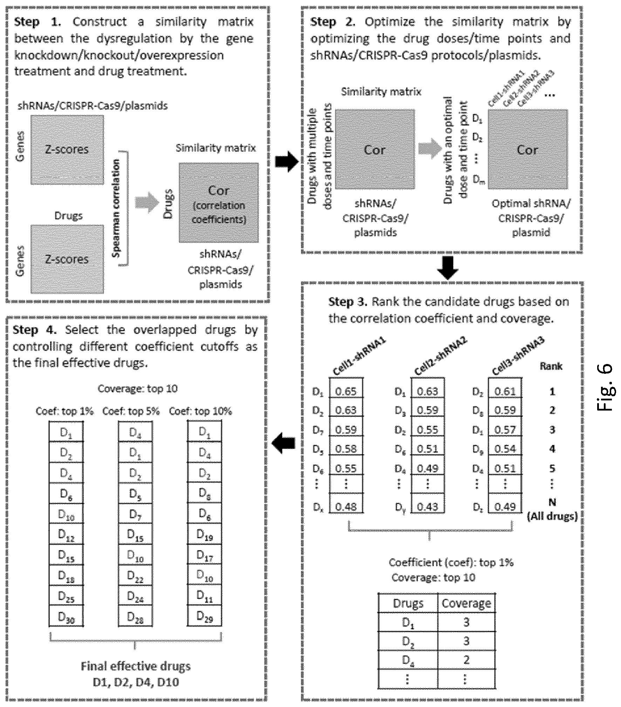

Fig 6 shows the flowchart of drug repositioning approach. Step 1) Extract the transcriptomic signature profiles associated with the gene knockdown, knockout, overexpression and drug treatment. Construct a similarity matrix between the molecular dysregulation induced by gene knockdown/knockout/overexpression treatment and drug treatment. Step 2) Optimize the similarity matrix by selecting the best drug dose, time point and shRNA/CRISPR-Cas9 protocol/plasmid. Step 3) Rank the candidate drugs based on the correlation coefficient and cell line coverage for each drug. Step 4) Select the overlapped drugs by controlling different coefficient cutoffs (Top 1 %, 5 % and 10 %) as the final drug candidates.

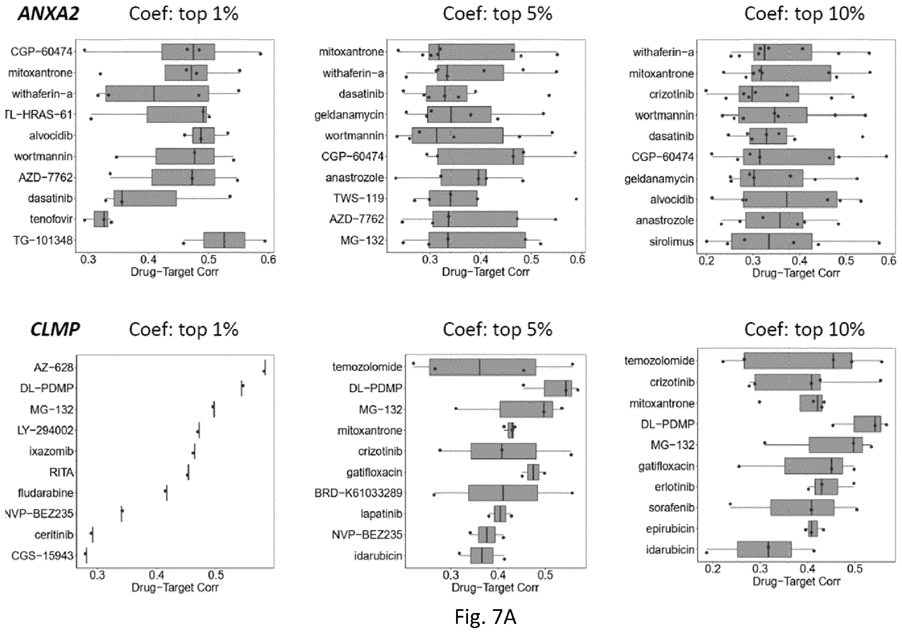

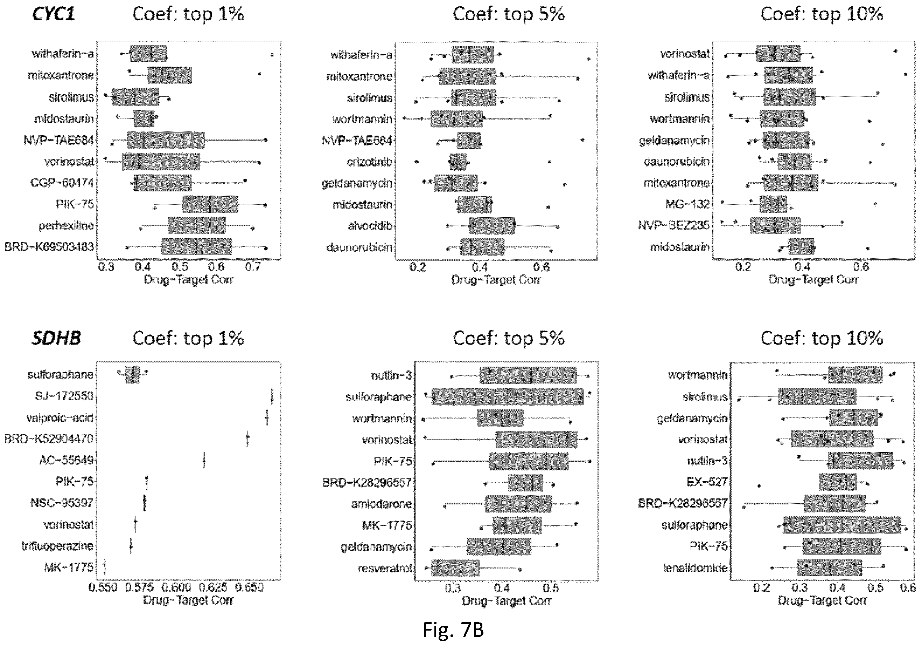

Fig 7 shows the box plots showing the top 10 drugs for targeting A) *ANXA2* and *CLMP,* B) *CYC1* and *SDHB,* by controlling the correlation coefficients within the top 1 %, 5 % and 10 %. Each point represents the similarity (calculated as Spearman correlation efficient) between the repurposed drug perturbed effects and gene knockdown/knockout/overexpression perturbed effect on a specific cell line. The bottom and top of the boxes represent the 25th and 75th percentiles. The whiskers represent the minimum and maximum values that are not outliers. The central band represents the median value.

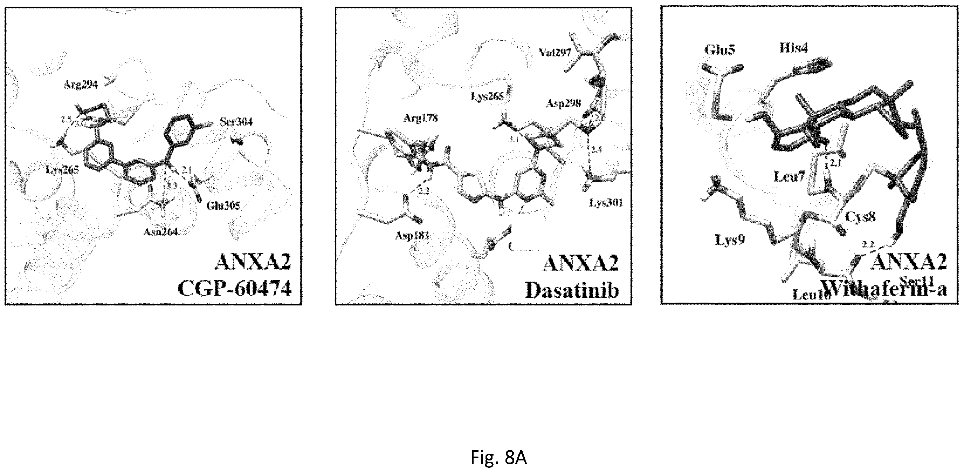

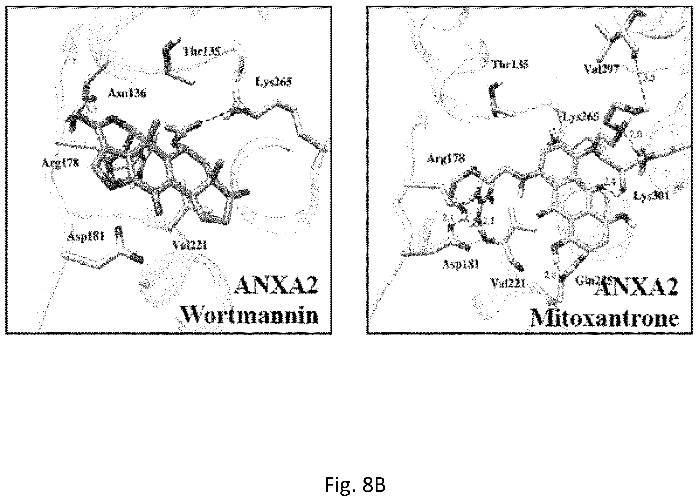

Fig 8 shows the graphical presentation of the binding mode of drugs with target gene proteins: A) ANXA2 - CGP-60474, ANXA2 - Dasatinib and ANXA2 - Withaferin-A, B) ANXA2 - Wortmannin and ANXA2 - Mitoxantrone. Black dotted lines represent the hydrogen bond interactions.

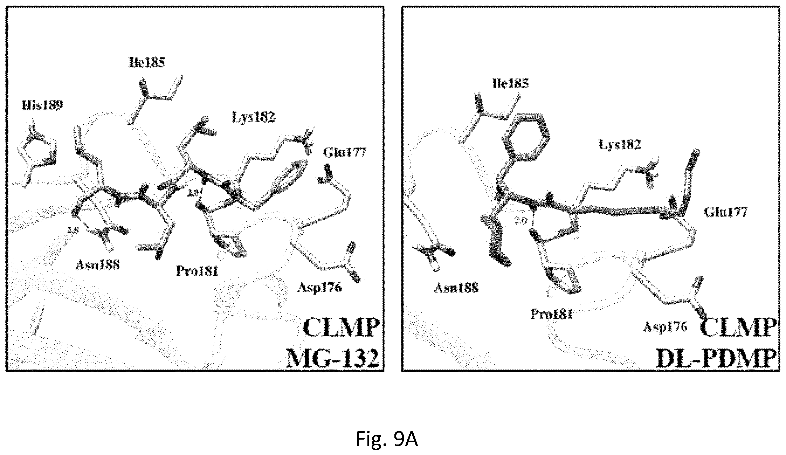

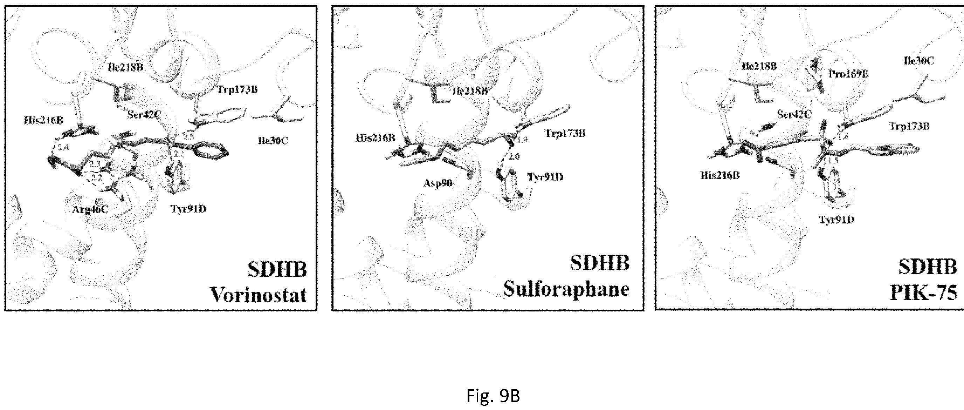

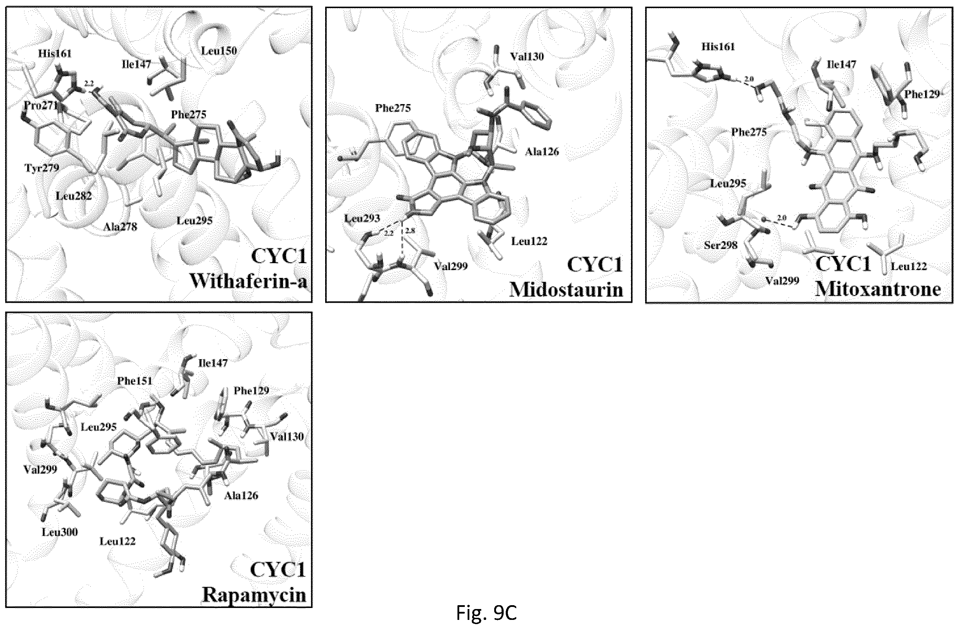

Fig 9 shows the graphical presentation of the binding mode of drugs with target gene proteins: A) CLMP - MG-132 and CLMP - DL-PDMP, B) SDHB - vorinostat, SDHB - sulforaphane and SDHB - PIK-75, C) CYC1 - rapamycin, CYC1 - withaferin-a, CYC1 - midostaurin and CYC1 - mitoxantrone. Black dotted lines represent the hydrogen bond interactions.

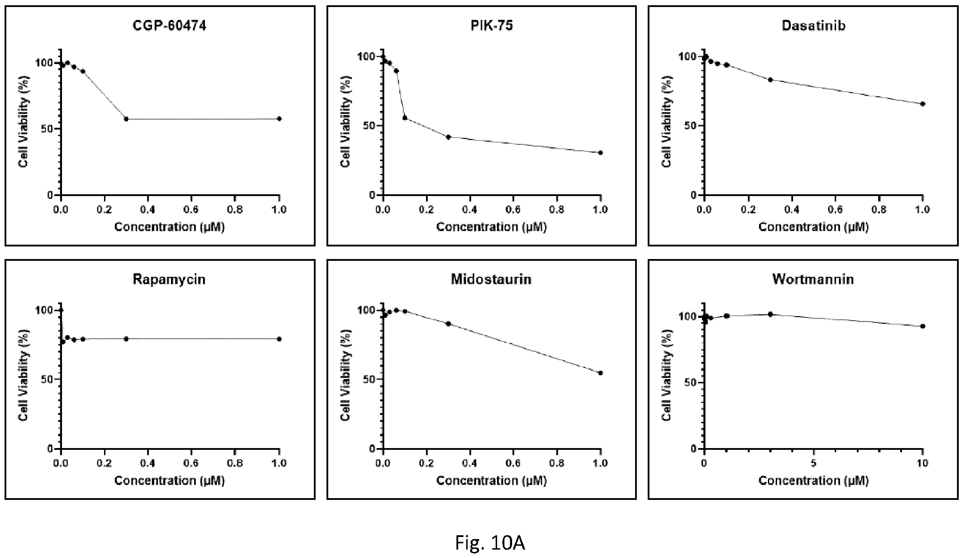

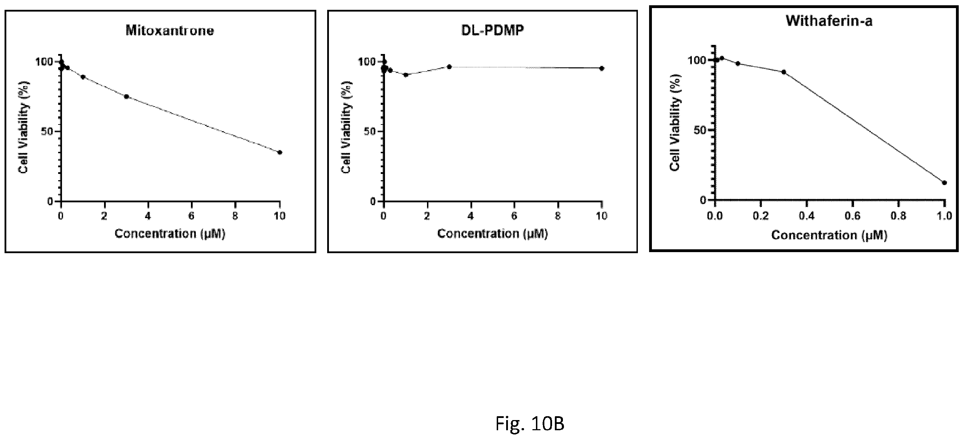

Fig 10 shows the cell viability in dose dependent manner performed by using CCK-8. A) CGP-60474, PIK-75, Dasatinib, Rapamycin, Midostaurin, Wortmannin. B) Mitoxantrone, DL-PDMP and Withaferin-A.

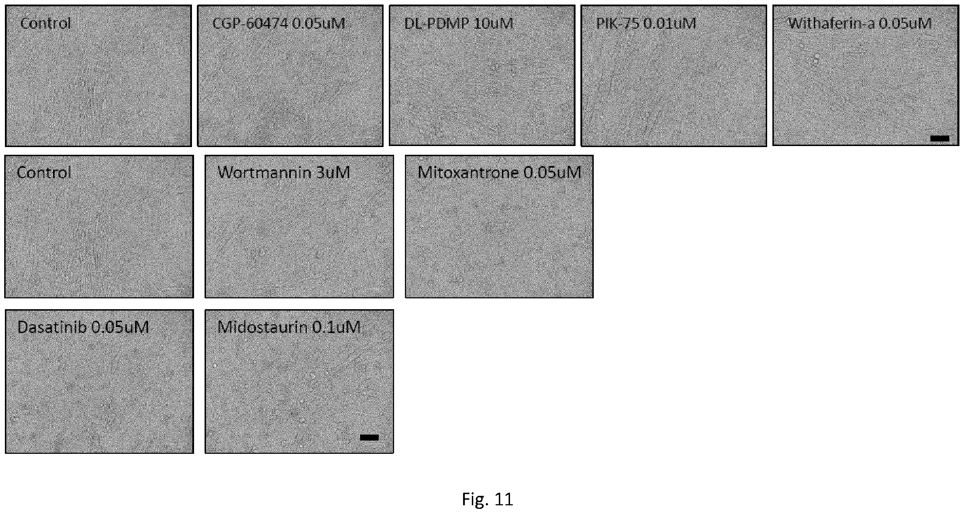

Fig 11 shows the phenotypic change with selected concentration of candidate drug during the cell viability assay. Scale bar: 100 µm.

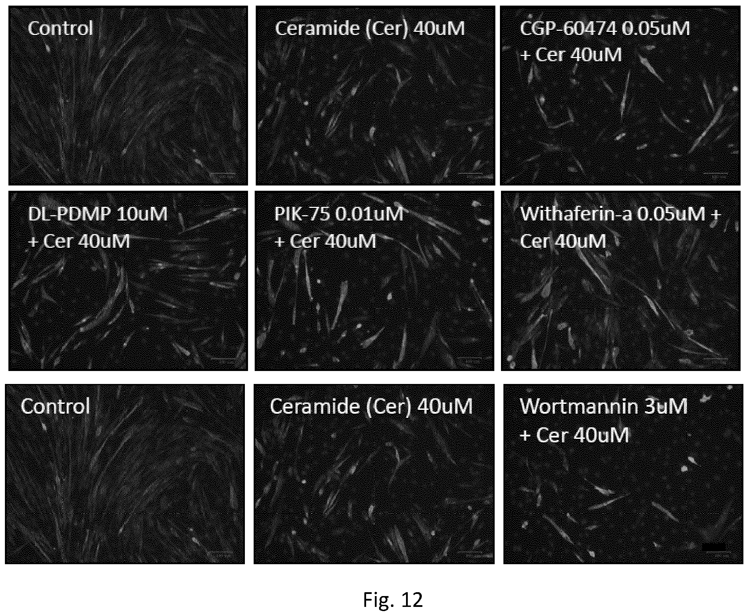

Fig 12 shows the phenotypic change with selected concentration of drug candidates in a *in vitro* sarcopenia model (40 µM of ceramide treatment).

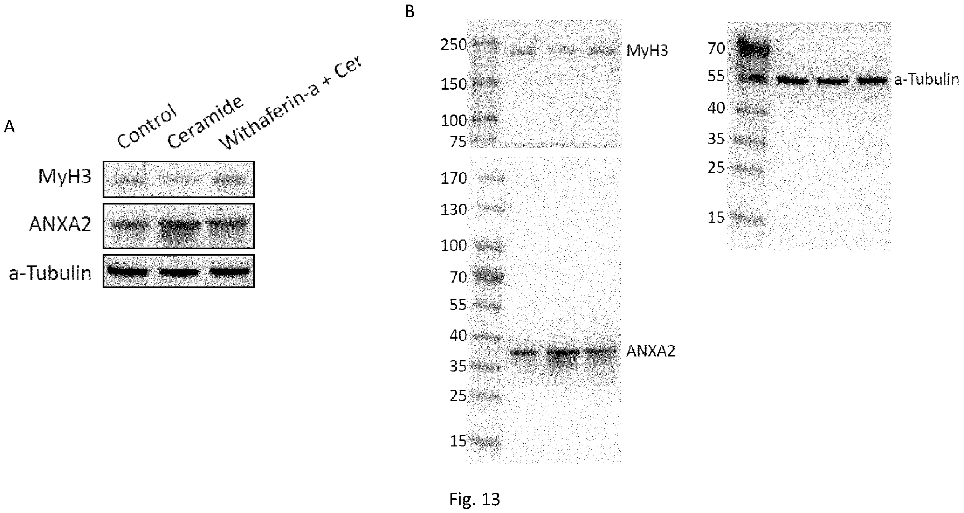

Fig 13 shows A) the protein expression level of MyH3 and ANXA2 of the sample that treated both of withaferin-a and ceramide. B) The uncropped western blot of protein expression level of MyH3 and ANXA2 of the sample that was treated with both of withaferin-a and ceramide.

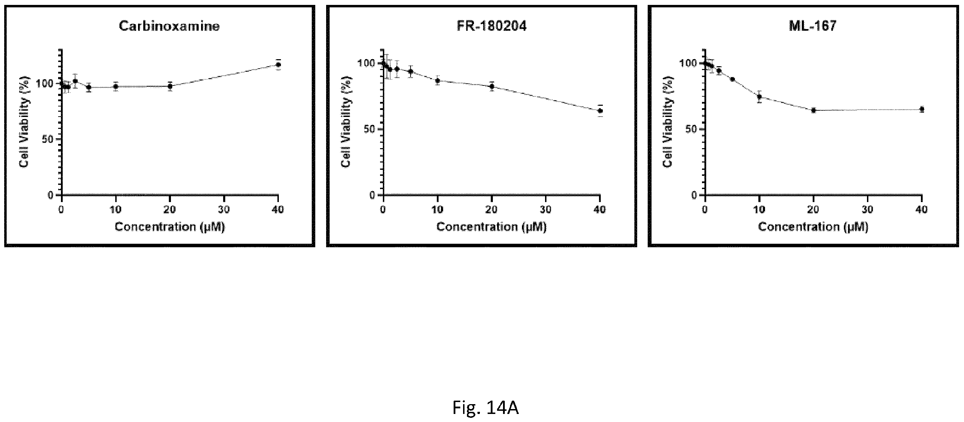

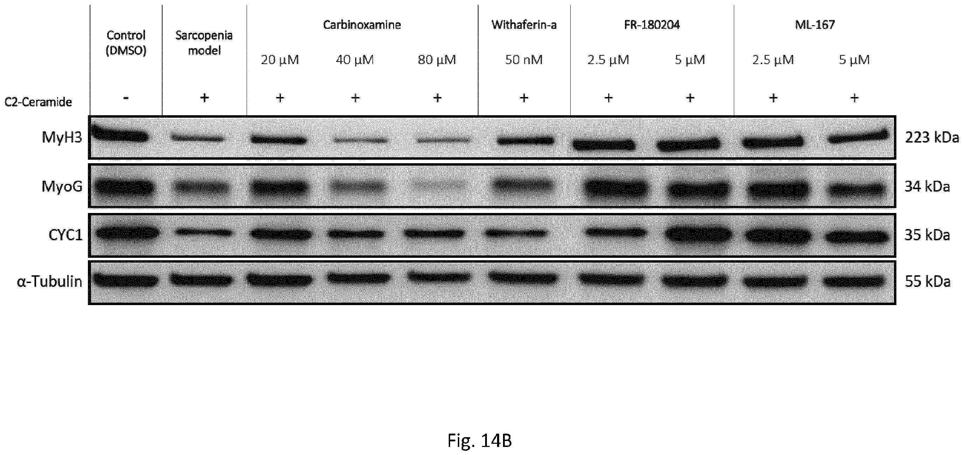

Fig 14 shows A) the cell viability in dose dependent performed by using CCK-8. B) The protein expression levels of MyH3, MyoG and CYC1 of the samples that were treated with carbinoxamine, withaferin-a, FR-180204 or ML-167 in the *in vitro* sarcopenia model.

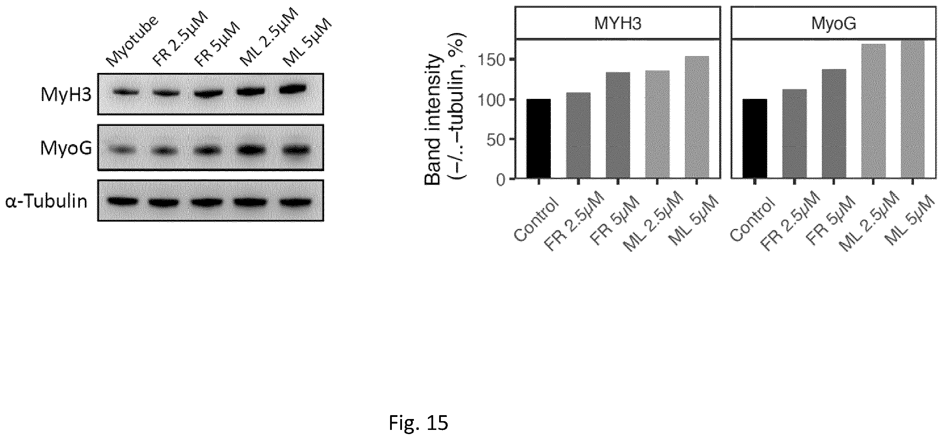

Fig 15 shows the protein expression levels of MyH3, MyoG of the samples that were treated with FR-180204 and ML-167 at two different concentrations, in a non-sarcopenia cell model (i.e., C2C12 cells not treated with C2-ceramide).

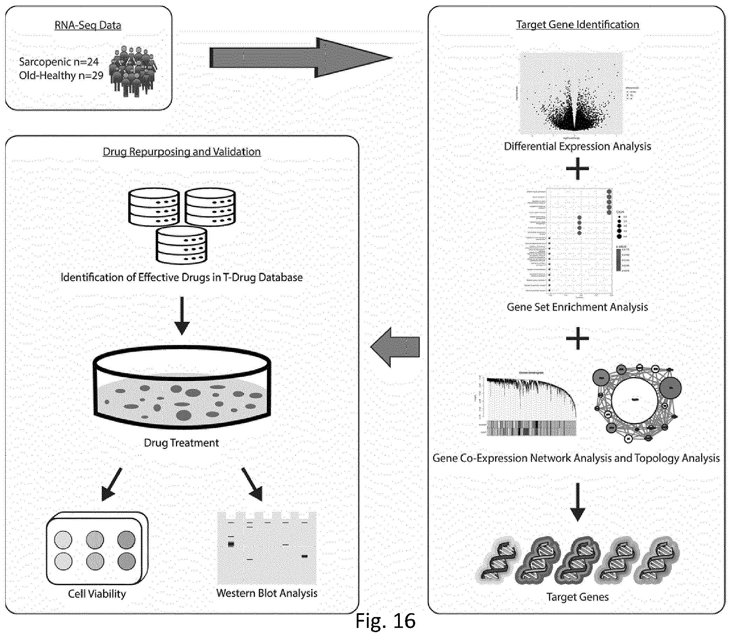

Fig 16 shows a diagram outlining a structured strategy of drug repurposing for the treatment of sarcopenia. RNA sequencing data and gene counts from a previous cohort were used to identify target genes. Differential expression analysis, gene set enrichment analysis, gene co-expression network analysis, and topology analysis were performed. A drug repositioning strategy was used to activate these genes and validated our findings with in vitro experiments.

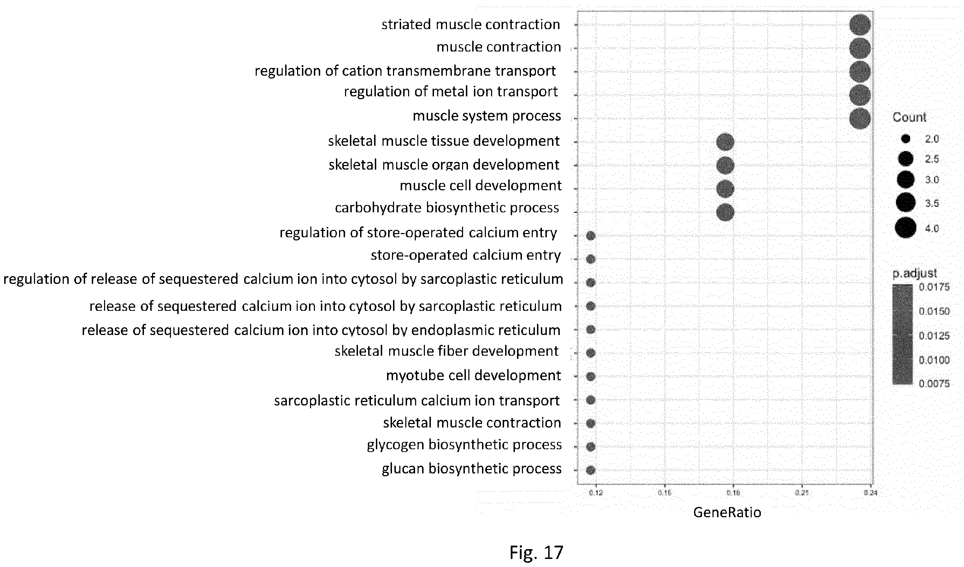

Fig 17 shows the top 20 enriched pathways of downregulated DEGs, based on the p-adjusted score. The dots' size represents the number of genes in a specific pathway. Among the downregulated differentially expressed genes (DEGs), the metabolic process of striated muscle contractions is the most frequently expressed.

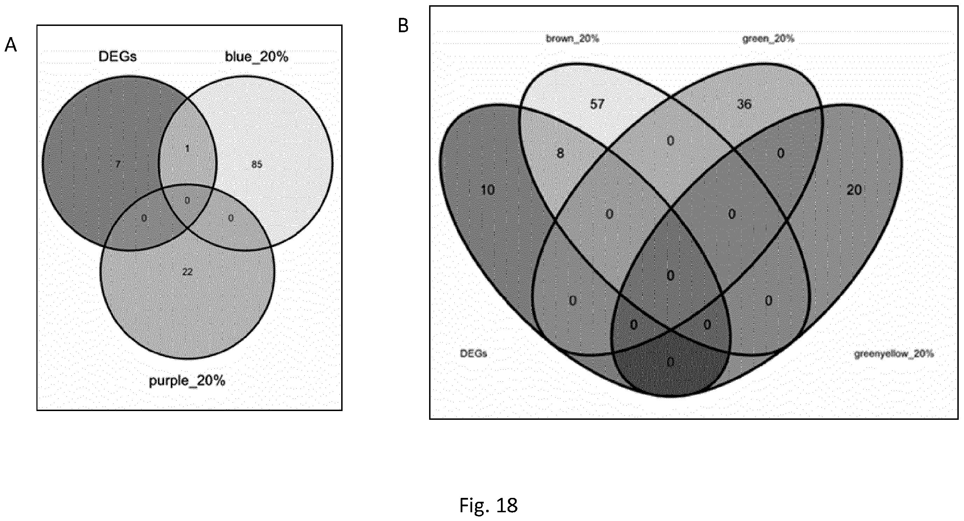

Fig 18 shows the intersection between the top 20 % genes of upregulated modules/upregulated DEGs (left) and the top 20 % genes of downregulated modules/downregulated DEGs (right). Two genes were discovered to be common between the upregulated modules and upregulated DEGs, whereas eight genes were found to be common between the downregulated modules and downregulated DEGs.

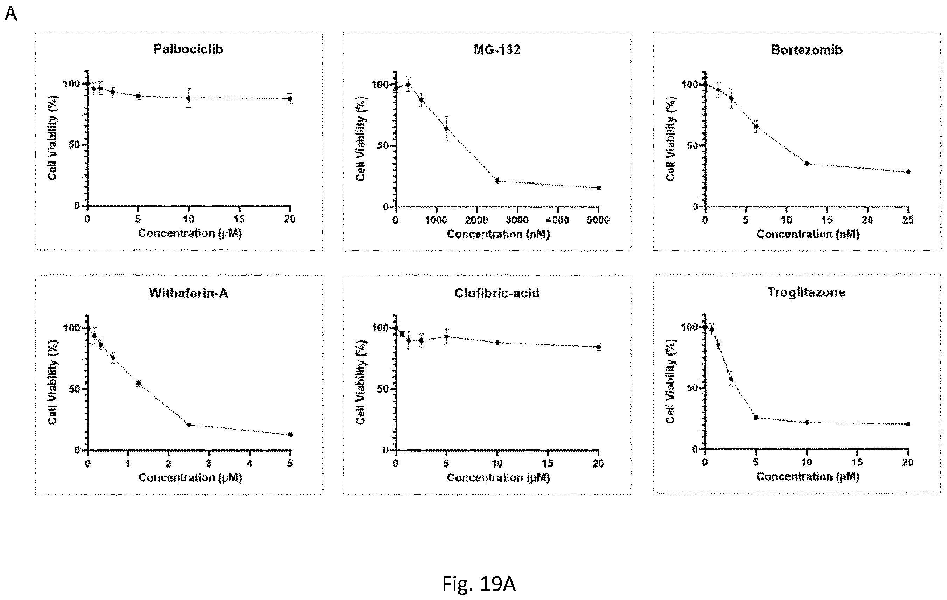

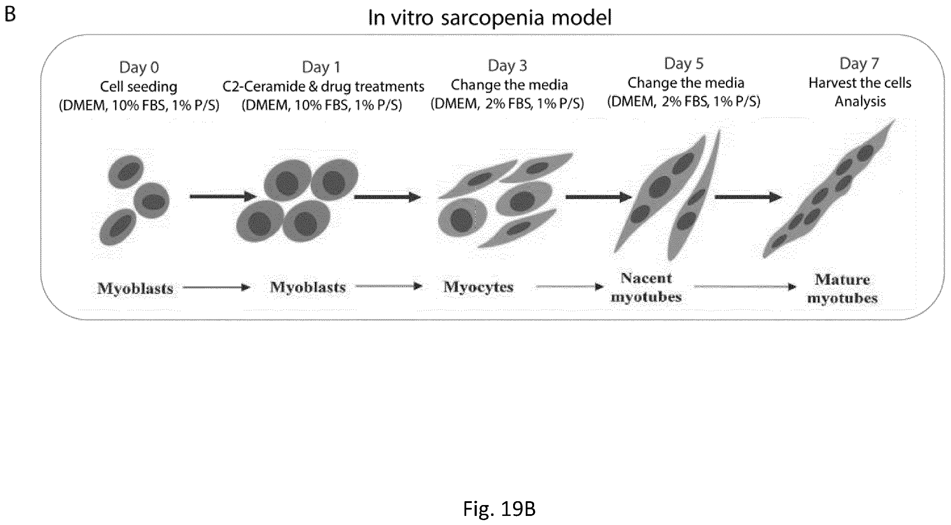

Fig 19 shows the efficacy of the target genes (AGL and PDHX) and the effect of the drugs on the activation of the target genes in the *in vitro* sarcopenia model. A) The toxicity of each drug as assessed by MTT assay. Each measurement was performed in triplicate. B) Induction of the *in vitro* sarcopenia model via C2-Ceramide in a C2C12 cell line and the stages of differentiation of myoblast cell to myotubes.

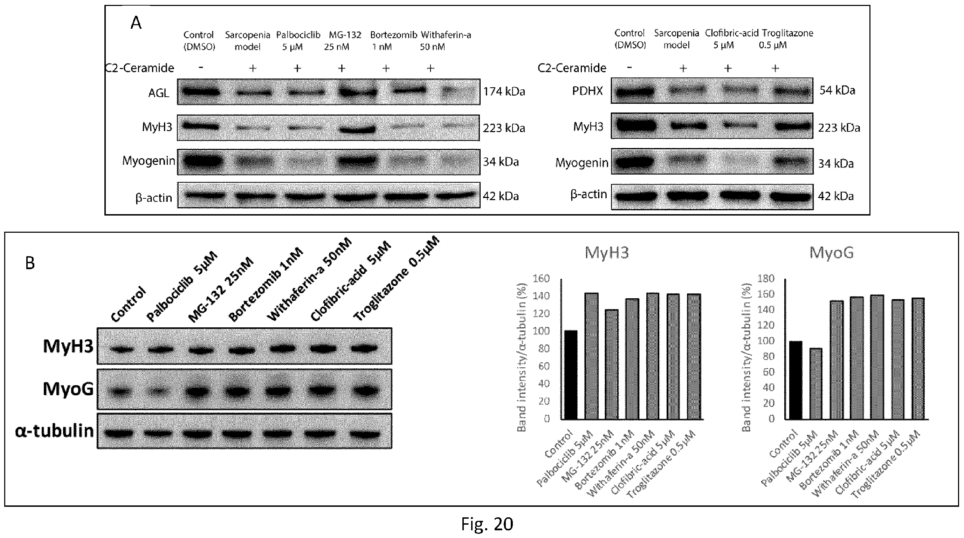

Fig 20 shows A) The activation of downregulated target genes (*AGL* and *PDHX*) via drugs with optimized doses as illustrated by Western blot. β-actin was used as a loading control. B) The expression of myogenic markers in cell models that were not exposed to ceramide, using drug optimized doses. α-tubulin was used as a reference protein during the analysis.

### DETAILED DESCRIPTION

Sarcopenia is defined as progressive muscle and function loss in the ageing population. It is an important public health problem which is causing falls, fractures, disabilities and death.

In this disclosure, the present inventors used systems biology to identify gene targets and effective drugs for use in the treatment of sarcopenia by integrating gene co-expression networks (GCN), differentially expressed genes (DEGs), gene ontology (GO), and gene perturbation expression profiles from various data sources. The effect of the target genes on phenotypes was validated in an *in vitro* sarcopenia model. Thus, six genes were identified as druggable target genes: *PDHX, AGL, ANXA2, CLMP, CYC1* and *SDHB. ANXA2, CLMP, CYC1* and *SDHB* encode the proteins involved in mitochondrial energy regulation and *PDHX* and *AGL* play essential roles in energy production and glycogen metabolism.

Muscle function relies heavily on the production of ATP for contraction and movement. The protein produced by the *CYC1* gene is involved in the transfer of electrons from the b-type cytochromes to cytochrome c, a key step in the mitochondrial electron transport chain which generates ATP [13]. As such, any disruption in the mitochondrial electron transport chain, such as mutations or dysregulation of the *CYC1* gene, could potentially impact muscle function [14]. These findings align with a recent study that reported a gene expression difference in elderly osteoporosis female patients based on the presence or absence of sarcopenia [15]. Among the 15 genes identified as differentially expressed in that study, CYC1 was also noted, further supporting the relevance of this gene in sarcopenia.

Annexins are calcium binding proteins that regulate various intracellular events that are altered in muscle diseases [16]. Recent *in vitro* and *in vivo* studies of myofibers and other cells have found that annexins and their binding partners are recruited to facilitate the repair of plasma membrane injury [17]. *ANXA1* and *ANXA2* interact with dysferlin to mediate sarcolemma repair and both of them have been reported to be upregulated in patients with dysferlinopathies [18]. Overexpression of *ANXA1* and *ANXA2* is likely an attempt to counteract the absence of dysferlin and restore cell membrane repair ability. It has been reported that the excess of *ANXA2* that leaks from injured myofibers activates muscle resident fibro/adipogenic precursors and differentiates into adipocytes, which gradually replace dysferlin-deficient myofibers leading to muscle degeneration [19]. Overexpression of the *ANXA2* is also observed in muscular dystrophies and shedding of ANX-positive vesicles has been shown in *ANO5*-knockout myofibers, suggesting these diseases may result from the fibrotic or adipogenic replacement of myofibers [18, 20]. An increase in the expression of *ANXA2* has also been reported in a rat model of desminopathy [21]. In this disclosure it is postulated that there is an enhanced requirement for phospholipid membrane repair in aging skeletal muscle and that the upregulation of *ANXA2* is, thus, a promising target against sarcopenic alterations.

*PDHX* gene encodes pyruvate dehydrogenase component X protein, which is necessary in order to sustain the functioning of pyruvate dehydrogenase and generate ATP [*22*]. Pyruvate dehydrogenase is a multi-enzyme complex situated in the matrix of mitochondria that catalyses the conversion of pyruvate to acetyl-coenzyme A. This is the initial irreversible step in the oxidation of carbohydrates [23]. Previous research showed a strong link between impaired mitochondrial function and sarcopenia [24]. Furthermore, deletion in the *PDHX* gene results in pyruvate dehydrogenase deficiency, lactic acidosis, and different levels of neurological disorders in clinical presentation [25].

Similarly, the *AGL* gene produces the glycogen-debranching enzyme, which is crucial in the process of breaking down glycogen [26]. It is a well-studied gene because its absence results in Glycogen Storage Disease Type III (GSD III) [27]. GSD III has 4 different subtypes, however, most individuals with the disease are deficient in the glycogen-debranching enzyme in both the liver and muscle (type IIIa) [28]. Clinical presentation of both GSD III and sarcopenia have similarities, such as muscle atrophy, weakness and peripheral neuropathy [29]. It is shown in this disclosure that defects in glycogen metabolism can be important in the pathophysiology of sarcopenia.

Accordingly, a compound for use in the treatment of sarcopenia is provided. The compound is selected from carbinoxamine, FR-180204, ML-167, withaferin-A, MG-132 and troglitazone.

In one embodiment, the compound is withaferin-A. It has been shown that treatment with withaferin-A is associated with an inhibitory effect on the expression levels of *ANXA2* and an activating effect on the expression levels of *CYC1.* Furthermore, treatment with withaferin-A is associated with an increase in expression levels of myogenic markers, MyoG and MyH3.

Withaferin-A is an active natural substance derived from the medicinal herb Withania somnifera and is known for its anti-inflammatory potential [30, 31]. Furthermore, in some studies withaferin-A has displayed attenuation in oxidative stress, and inhibiting cell proliferation based on *in vitro* or *in vivo* studies [30]. Withaferin-A has also shown effects on inducing apoptosis and evading proliferative signals within a tumour microenvironment [32].

It has previously been shown that withaferin-A can inhibit the NF-κB signalling pathway and that NF-κB inhibitors can improve the muscle function in the mdx mice which is a popular model for Duchenne muscular dystrophy [33]. Thus, in another embodiment, the method of treatment comprises administration of withaferin-A together with at least one NF-κB inhibitor. The combination of withaferin-A and an NF-κB inhibitor generates a synergistic effect on the improvement of skeletal muscle cells. An example of a NF- κB inhibitor is bortezomib, a proteasome inhibitor that prevents NF-κB activation by inhibiting the degradation of IκBα, the inhibitor of NF-κB.

In one embodiment, the compound is carbinoxamine. Treatment of sarcopenic cells with carbinoxamine has been shown to increase the expression levels of *Cyc1,* MyoG and MyH3. Carbinoxamine is an antihistamine and has previously demonstrated potential in mitigating inflammation and oxidative stress in muscle tissue [34].

In one embodiment, the compound is FR-180204. Treatment of sarcopenic cells with FR-180204 has been shown to significantly increase the expression levels of *Cyc1,* MyoG and MyH3. FR-180204 has previously been shown to inhibit extracellular signal-regulated kinases (ERK), which plays a role in muscle protein synthesis [35].

In one embodiment, the compound is ML-167. Treatment of sarcopenic cells with ML-167 has been shown to significantly increase the expression levels of *Cyc1,* MyoG and MyH3. ML-167 has been shown to selectively inhibit the Cdc2-like kinase 4, which is implicated in muscle atrophy [36].

Furthermore, FR-180204 and ML-167 also have the ability to improve the myotube fusion in healthy muscle myoblasts.

In one embodiment, the compound is MG-132. Treatment of sarcopenic cells with MG-132 has been shown to significantly increase the expression levels of *AGL,* MyoG and MyH3. MG-132 is a proteasome inhibitor that suppresses the ubiquitin-proteasome pathway [37]. This pathway plays a significant role in muscle atrophy, as skeletal muscles undergo atrophy under various conditions, and myofibrillar proteins are degraded through the ubiquitin-proteasome system [38]. Recent research has provided evidence supporting the effectiveness of MG-132 in mitigating muscle atrophy [39]. Sakai et al. observed increased expression levels of ubiquitinated proteins in atrophic muscle cells and their downregulation after introducing MG-132 in mice [39].

In one embodiment, the compound is trolitazone. Treatment of sarcopenic cells with trolitazone has been shown to significantly increase the expression levels of *PDHX,* MyoG and MyH3. Troglitazone belongs to the family of oral antidiabetic drugs and is known for its ability to reduce insulin resistance and serum triglyceride levels [40]. A rodent study conducted by Unger and Orci has also highlighted its potential in improving myocardial contractility in cases of cardiomyopathy [41]. They found that troglitazone administration resulted in increased myocardial contractility in cardiomyopathic rats.

The effects of treatment with the above drugs on expression levels of target genes and myogenic markers has been observed at non-toxic concentration, i.e., no or little influence on cell viability has been observed at the tested drug concentrations.

### EXAMPLES

### Example 1

This example pertains to drug development based on the interaction with the target genes *ANXA2, CLMP, CYC1* and *SDHB,* see Fig. 1.

### Methods

### Data source and pre-processing

The RNA sequencing data of muscle biopsies of elderly individuals from Singapore (SSS, 40 male participants aged 65-79 years old), Jamaica (JSS, 39 male Afro-Caribbean participants aged 63-89 years old), and Hertfordshire (HSS, 40 male Caucasian participants aged 68-76 years old) cohorts were generated in a previous study [42]. Raw fastq files were downloaded from the NCBI SRA database (https://www.ncbi.nlm.nih.gov/sra) and processed by using Kallisto pipeline. In total, 931 samples from 119 patients (i.e., 7 or 8 biopsy samples per patient) were analysed, and the mean value of transcripts per kilobase million (TPM) was used as the final gene expression level for each patient. For each dataset, the genes with annotation of "protein-coding gene" were reserved, and then the low-expressed genes (average TPM < 1) were trimmed off in the downstream analysis.

### Determination of differentially expressed genes (DEGs)

The DEGs between sarcopenia and non-sarcopenia patients were retrieved by performing gene differential analysis using DESeq2 package (v1.32.0), and Gene Ontology (GO) analysis using *clusterProfiler* package (v4.0.0) was performed to reveal the biological function of sarcopenia by controlling p value < 0.05.

### GCN analysis and module identification

Spearman correlation was used to estimate the correlation between each two genes across all samples. The gene-gene links whose positive correlations ranked in the top 10% with FDR<0.05 were retrieved to construct the sarcopenia GCNs. The Leiden algorithm was employed to detect the optimal module partition. To get significant functional analysis, only modules consisted of more than 30 were extracted for further analysis. The GCN identification was performed with Pycharm (Version-2020.2.5) with packages *SciPy* [43] and *leidenalg* (v0.7.0) and visualized using Cytoscape-v 3.8.2 [44].

### Concordance analysis and topology analysis

Concordance analysis was performed to measure the module regulate attributes. A module is denoted as upregulated or downregulated module if it has a significant overlap with the upregulated or downregulated DEGs, respectively, between sarcopenia and non-sarcopenia samples (hypergeometric test, FDR<0.05).

To obtain insights of specific gene influence among the modules, topology analysis was performed by integrating three centrality parameters: degree, betweenness and closeness. The top 20% ranking genes shared based on the three parameters were identified as the hub genes for the module. The shared hub genes among the modules in all the three cohorts were regarded as final candidate target genes for sarcopenia.

The *Enrichr* function from the python package *gseapy* (version 0.9.16; https://github.com/zqfang/gseapy) was implemented to identify the enriched GO biological progress for the gene modules (FDR<0.05). All GO terms were obtained from the database source of *Enrichr* [7].

### Drug repositioning

The transcriptomic signature profiles associated with shRNA knockdown, CRISPR-Cas9 knockout, plasmid overexpression and chemical perturbation for human cell lines were downloaded from CMap LINCS 2020 (level 5 data, updated in 2021/6/17, https://clue.io/data/CMap2020#LINCS2020). The level 5 data provides robust moderated z-score (differential expression values) of genes by at least 3 times replicate biological experiments. As described previously [45], a drug repositioning method was developed based on the shRNA knockout/CRISPR knockout/plasmid overexpression and chemical perturbation profiles integration, with the hypothesis that a drug has a repressed (or activated) effect on the expression of a target gene in muscle cells if the drug treatment leads to a similar dysregulation on gene expression induced by this target gene knockdown or knockout (or overexpression) in cell lines. This approach was applied for the drug repositioning analysis in sarcopenia. In brief, there were four steps:
1) Construct a similarity matrix between the molecular dysregulation induced by gene knockdown/knockout/overexpression treatment and drug treatment. Firstly, the knockdown/knockout/overexpression/drug-perturbed level 5 differential expression profiles associated with each target gene were extracted and denoted as the perturbed-specific signature matrixes. Since there was no muscle cell line in the CMap data resource, the perturbed-specific signature profiles from 15 available human cancer were downloaded and normal tissue cell lines associated with the predicted target genes, based on the hypothesis that the binding effect between the drug and its target protein is independent of cell types if the drug could be transported into cells. Among the 15 cell lines, the *ANXA2* knockdown by shRNA was associated with thereinto 8 cell lines (A375, A549, HA1E, HCC515, HEPG2, MCF7, PC3 and VCAP), *CLMP* knockout by CRISPR-Cas9 technology in 10 cell lines (A375, A549, AGS, BICR6, ES2, HT29, MCF7, PC3, U251MG, and YAPC), *CYC1* overexpression by plasmids in 9 cell lines (A375, A549, HA1E, HCC515, HEPG2, HT29, MCF7, PC3 and VCAP) and *SDHB* overexpression by plasmids in 10 cell lines (A375, A549, H1299, HA1E, HCC515, HEPG2, HT29, MCF7, PC3 and VCAP). Meanwhile, 3887, 118, 3887 and 356 drugs for the above cell line sets were collected, respectively. Further, for each of the cell line regarding a specific target gene, a similarity matrix was generated by calculating Spearman correlation between all possible combinations of drug-perturbed signature profiles and target-specific knockdown/knockout/overexpression-perturbed signature profiles. The coefficients in this matrix represent the similarity of effects on gene expression induced by specific shRNA knockdown/CRISPR-Cas9 knockout/plasmid overexpression and drug treatment. In this matrix, each row represents a drug with specific dose and treated time and each column represents a specific shRNA, CRISPR-Cas9 protocol or plasmid.
2) Optimize the similarity matrix. In the experiment setting of CMap, different shRNAs, CRISPR-Cas9 protocols or plasmids were used for targeting the same gene in each cell line. Considering this, the median correlation coefficients were calculated across all drug setting for each shRNA, CRISPR-Cas9 protocol or plasmid and only kept the shRNA, CRISPR-Cas9 protocol or plasmid with the highest median in a specific cell line. In addition, the different doses and time points for each drug were set in the drug treatment experiments, which may induce different effects on the gene expression in cells. Considering this, the strongest dose and time point for every drug whose dysregulation on gene expression showed the highest correlation coefficient with the dysregulation induced by the target gene knockdown/knockout/overexpression was kept. As a result, an optimized correlation matrix for each target was generated in which each row represented a drug, and each column represented an optimal shRNA/CRISPR-Cas9 protocol/plasmid in a specific cell line.
3) Rank the candidate drugs based on the correlation coefficient. Within each cell line, all the drugs were ranked based on an increasing order of correlation coefficient. For each target gene, the drugs which ranked within top 1% based on the highest correlation coefficients in each cell line were extracted and this was repeated in all different cell lines. A union drug list across all cell lines was obtained and the coverage of each drug was calculated which indicates the number of cell lines where the drug could potentially inhibit or activate the expression of the specific target gene. In this drug list, the top 10 drugs with the highest coverages were chosen. This process was repeated to obtain another two top-10-drug list considering 5 % and 10 % similarity coefficient in each cell line.
4) Select the most effective drugs for each target gene. Based on step 3, lists of potentially effective drugs (each with 10 drugs) by the different coefficient cutoffs 1 %, 5 % and 10 % were obtained. The overlapped drugs among the three lists were selected as the final most effective drugs.

### Molecular Docking

The crystal structure of the Annexin A2 (ANXA2; PDB ID: 2HYV) was retrieved from the RCSB protein data bank [46]. The FASTA sequences were retrieved from UniProt with accession codes: Q9H6B4, P31930 and P21912 for the structure of CXADR-membrane like protein (CLMP), Cytochrome C1 (CYC1 c1) and Succinate dehydrogenase iron-sulfur subunit (SDHB), respectively. The homology model of these structures was modelled using SWISS MODEL webserver [47]. However, missing parts of the starting structures were constructed using MOE software (Molecular Operating Environment, v2019.01). The overall quality of the developed models and their stereochemical properties were assessed by the PROCHECK Ramachandran plot [48]. Verify 3D [49] was used to compute the compatibility of the 3D model with its amino acid sequence, whereas, ERRAT plot [50] was used to assess the distribution of atoms with respect to one another and their non-bonded atomic interactions. The Site Finder program in MOE was used to detect potential binding sites in the modelled structures. This technique detects α shapes on the protein structures and assesses them based on the tendency of the compound's binding (aka propensity of ligand binding or PLB). The site with the highest PLB score was used for further docking studies. Compounds partial charges were derived with the MMFF94x force field [51]. All stabilizing agents, water molecules present in the crystal structures were removed. The protonate 3D module in MOE was used for assigning the protonation state to every titratable residue at physiological conditions within the complexes. MOE docking suite was employed for docking studies. The standard default settings were used with London dG scoring function as a fitness function and were used to evaluate a hundred conformations. Then these conformations were further refined to thirty conformations by Generalized Born Volume Integral/Weighted Surface area scoring function (GBVI/WSA) Δ*G* with the induced-fit protocol. Conformations with good GBVI/WSA scores were considered for further studies.

### In vitro validation

The C2C12 cells (C2C12 mouse C3H muscle myoblast purchased from Sigma) were maintained in modified Eagle's medium (DMEM) with 10 % fetal bovine serum (FBS) and 1 % penicillin/streptomycin (P/S) solution in a humidified incubator with 5 % CO₂ at 37 °C. 40 µM C2-ceramide was used for developing an in *vitro* model of sarcopenia in the C2C12 cell line, i.e., *in vitro* sarcopenia model. One day after 300×103 cells were seeded per well in a 6-well plate, N-acetyl-D-sphingosine (C2-ceramide) and the drug candidates (dissolved in DMSO) were administered in the maintenance condition (DMEM with 10 % FBS and 1 % P/S) for two days. Then, the medium was changed twice, at two-day intervals, with a differentiation medium (DMEM with 2% FBS and 1% P/S) with drug candidates to differentiate myoblasts into myotubes. Furthermore, siRNAs for *Anxa2* and *Clmp* and overexpression plasmids for *Cyc1* and *Sdhb* were transfected into the cells to examine the effect of the target proteins on the *in vitro* sarcopenia model. Cell viability was measured spectrophotometrically by Cell Counting kit-8. The expression level of target proteins (*ANXA2, CLMP, Cyc1, and SDHB*), myogenic markers (MyoG and MyH3) and α-Tubulin (internal control) were detected using Western blots. All experiments were performed with 3 replicates.

### siRNA and plasmid transfection

First day after seeding, 1 pmol of siRNAs for targeting *ANXA2* and *CLMP* were transfected into the C2C12 cells by using the Lipofectamine^{®} 2000 RNAiMAX reagent for 2 days. 5 µg/µl of DNA plasmids of *CYC1* and *SDHB* were transfected into the C2C12 cells by using the Lipofectamine^{®} 3000 reagent for 2 days. The 40 µM of C2-ceramide was treated right before transfection. The photos and proteins were taken after 4 days of differentiation.

### Cell viability assay

Cell proliferation was detected by Cell Counting kit-8 (CCK-8) assay. 10×103 cells were seeded per well in a 96-well plate. The drugs were treated the day after seeding in the 96-well plate. Following a 24 h treatment period, 10 µl of CCK-8 reagent was added to each well and the cells were incubated at 37 °C for 2 hours. Absorbance was measured at 450 nm by using a microplate reader (Hidex Sense Beta Plus).

### Immunofluorescence staining

After 3 days of differentiation, cells were washed 3 times in PBS and then fixed in 4 % paraformaldehyde solution for 10 min at 37 °C. The cells were, once again, washed 3 times in PBS and then permeabilized in 0.1 % TritonX-100 for 15 min at room temperature. The cells were then incubated for 1 h in 2 % bovine serum albumin (BSA) solution followed by overnight incubation in anti-Myosin/MyH3 diluted in 0.1 % BSA at 4 °C. Primary antibody solution was washed away with PBS, then incubated in fluorescent dye-labelled secondary antibody diluted in 0.1 % BSA for 45 min at room temperature protected from light. Finally, the samples were washed 3 times with 1xPBS-T and mounted with DAPI mount putted cover glass on the cells.

### Quantification of MyH and intensity

The cell brightness was measured by ImageJ software (NIH). For each experiment condition, three different areas were photographed with same area size (5038848 by ImageJ), the mean brightness of each image was obtained for statistical analysis. The t test was used to check the statistical differences between any two experiment conditions.

### Western blot analysis

The cells were washed with PBS and lysed with CelLytic M (C2978, Sigma-Aldrich) lysis buffer containing protease inhibitors. The cell lysates were centrifuged at 12 000 rpm for 5 min and supernatants were collected. A protein assay solution (Bio-Rad) was used for the determination of protein concentration. The absorbance of the proteins was measured spectrophotometrically at 595 nm by microplate reader (Hidex Sense Beta Plus). Protein electrophoresis was performed using Mini-PROTEAN^{®} TGXTM Precast Gels (Bio-Rad) and the separated proteins were transferred to a Trans-Blot Turbo Mini 0.2 um PVDF Transfer Packs membrane (Bio-Rad) by using Trans-Blot^{®} TurboTM Transfer System (Bio-Rad). The membrane was blocked with 5 % skim milk for 30 min at 4 °C with gentle rocking. It was immunoblotted with primary antibodies against MyH3, MyoG, *ANXA2, CLMP, Cyc1,* and *SDHB,* as well as α-Tubulin, as an internal control, overnight at 4 °C with gentle rocking. Goat anti-Rabbit HRP or goat anti-mouse IgG-HRP was used as secondary antibody. Reaction with secondary antibody proceeded for 30 min at 4 °C with gentle rocking. The protein bands on the membrane were revealed using enhanced chemiluminescence substrate (ECL, Bio-Rad) and detected with ImageQuanattm LAS500 (29-0050-63, GE).

### Results

### Muscle transcriptomics analysis reveals sarcopenia-specific gene signatures

Differential expression analysis on Singapore cohort (SSS cohort) was performed and revealed the transcriptomic alterations in the muscle of sarcopenia patients compared to the control subjects with no sarcopenia. 20 sarcopenic and 20 non-sarcopenic samples in the SSS cohort were analysed, which is a balanced sample size for differential expression analysis. As a result, 667 DEGs in the SSS cohort (p-value < 0.05) were identified, where 256 genes were upregulated, and 411 genes were downregulated in the sarcopenic samples compared to the control samples, see Fig. 2A. Based on the functional enrichment analysis, the upregulated DEGs were significantly enriched in the skin ageing-related pathways, including epidermis cell differentiation, cornification and keratinocyte differentiation. These processes are commonly observed in aging cells, leading to the weakness of the water-proof barrier [52]. The downregulated DEGs were significantly enriched in muscle bioenergetics related processes, including oxidative phosphorylation, ATP synthesis, and mitochondrial dysfunctions, see Fig. 2A. As a main energy-related organelle, mitochondrial-involved biological processes have been proven to be decreased regarding ATP production rate (coupled with oxidative phosphorylation) [53], mitochondrial electron transport [54] and respiratory [55].

### GCN analysis identifies sarcopenia-specific functional modules

GCN analysis is a powerful tool for identifying key gene clusters, which drive the disease progression and occurrence. GCNs based on the gene expression profiles of SSS and the other two independent sarcopenia cohorts, including JSS and HSS cohorts, were constructed. In brief, the Spearman correlation coefficient between each possible gene pair was calculated based on their mRNA expression levels and the gene-gene links whose correlation coefficient ranked within the top 10 % were extracted to construct the GCN. 150 708, 1 236 631 and 150 054 gene-gene links were obtained with correlation coefficients ranging from 0.67, 0.71 and 0.67 to 1 in the HSS, JSS and SSS cohorts, respectively. By using the Leiden algorithm module identification method, eight (C0-C7, 6735 genes), six (C0-C5, 8269 genes) and eight (C0-C7, 7692 genes) modules were found with more than 30 nodes in HSS, JSS and SSS cohorts, respectively. Then, the association of each module with the upregulated and downregulated sarcopenia signature genes was investigated by concordance analysis. As a result, it was found that the 875 genes in HSS-C1 had a significant overlap with the 251 upregulated sarcopenia signature genes (n=35, hypergeometric test, FDR<0.05). Thus, it was found that HSS-C1 has an upregulated module in sarcopenia, Fig. 2B. Meanwhile, the genes in HSS-C3 (596 genes) and HSS-C5 (515 genes) had significant overlaps with the 390 downregulated sarcopenia signature genes, respectively (n=125 and 39, hypergeometric test, FDR<0.05), which were determined as the downregulated modules, see Fig. 2B. Similarly, JSS-C1, JSS-C4, SSS-C1, SSS-C2, SSS-C4 and SSS-C7 were identified as the upregulated modules, whereas JSS-C2, SSS-C3 and SSS-C5 were identified as the downregulated modules in the JSS and SSS cohorts, respectively, see Fig. 2B.

Next, Gene Ontology (GO) functional enrichment analysis based on the genes in the identified modules was performed to investigate their functional biological properties. Interestingly, it was observed that most of the modules could be grouped based on five categories of biological processes, see Fig. 2B. The genes in the three upregulated modules, including HSS-C1, JSS-C1 and SSS-C2, were significantly enriched in the cell migration, cell proliferation, cell-differentiation, cell-matrix adhesion and immune response pathways. Moreover, the genes in the eight modules HSS-C0, HSS-C7, JSS-C0, JSS-C3, JSS-C5, SSS-C0 and SSS-C6 were significantly enriched in the DNA/RNA processing and protein transport pathways, including DNA replication, DNA repair, mRNA/rRNA/tRNA processing, protein localization to the membrane, protein targeting to ER, and protein ubiquitination. The genes in the three downregulated modules, HSS-C3, JSS-C2 and SSS-C3, were significantly enriched in the energy metabolism and mitochondrial dysfunction, including oxidative phosphorylation, ATP synthesis, and mitochondrial electron transport, which is consistent with previous research implicating a dysregulated mitochondrial energy metabolism in the pathogenic and/or development process of sarcopenia [54-56]. In addition, the genes in HSS-C4, JSS-C4, SSS-C4 and SSS-C7 were significantly enriched in muscle-related terms, including muscle fibrosis and muscle contraction, and three of these modules were upregulated modules. Finally, two downregulated modules (HSS-C5 and SSS-C5) were significantly enriched in the angiogenesis pathway, suggesting a dysregulated vascular development in sarcopenia progression.

### Identification of target genes for treatment of sarcopenia

To identify the consensus gene modules, all upregulated and downregulated modules among HSS, JSS and SSS cohort pairs were compared, see Fig. 3A. As a result, three significantly overlapped (P<0.05) upregulated module groups: HSS-C1 (n = 875), JSS-C1 (n = 2058) and SSS-C2 (n = 1415) and three significantly overlapped downregulated modules: HSS-C3 (n = 596), JSS-C2 (n = 1877) and SSS-C3 (n = 1011) were found. Notably, 401 genes in upregulated clusters were overlapped, see Fig. 3B, enriched in collagen fibril organization, regulation of cell migration and angiogenesis. In addition, 298 genes were shared by downregulated clusters, see Fig. 3B, enriched in cell bioenergetic processes, including oxidative phosphorylation, mitochondrial ATP synthesis and respiratory electron transport chain.

Next, network topology analysis was performed by evaluating the centrality (degree, betweenness and closeness parameters) of the genes in each of the overlapped upregulated and downregulated modules. All genes in each of the upregulated modules (HSS-C1, JSS-C1 and SSS-C2) were sorted according to the descending order of each centrality parameter and the top 20% of genes in each module were extracted. As a result of the intersection of the three centrality parameters, 123 top genes from HSS-C1, 247 top genes from JSS-C1 and 195 genes from SSS-C2 were extracted. Finally, three upregulated gene targets (*ANXA2, CLMP* and *CYBRD1*) were found overlapping between these upregulated module genes and upregulated signature genes, see Fig. 3C. Similarly, 52 top genes from HSS-C3, 220 top genes from JSS-C2 and 109 genes from SSS-C3 were extracted. Finally, four downregulated gene targets (*CYC1, ATP5F1B, SDHB* and *COX8A*) were found overlapping these downregulated module genes and downregulated signature genes, see Fig. 3C. These seven genes were identified as final druggable targets that could potentially be used in the treatment of sarcopenia.

Next, drug repositioning was performed for these target genes and the CMap data resource (https://clue.io/data/CMap2020#LINCS2020) was examined. *ANXA2, CLMP, CYC1* and *SDHB* were used as candidate target genes for sarcopenia drug repositioning since the other target genes' knockdown information was not available in the CMap data resource. Previous studies have shown that *ANXA2* is a critical member of the annexin family of proteins, and it is involved in a variety of cellular functions, including vesicle transport, cell division, calcium signalling and cell growth [57]. *CLMP* is a type I transmembrane protein involved in adipocyte maturation, germ cell translocation and small intestine development [58]. *CYC1* encodes a subunit of the cytochrome bc1 complex [59] and *SDHB* encodes a subunit involved in the oxidation of succinate [60]; both genes play essential roles in the mitochondrial respiratory chain.

### Target genes can induce myotubes differentiation based on cell experiments

To validate the predictions based on the transcriptomics data of the sarcopenia, an *in vitro* sarcopenia model was developed wherein a C2C12 cell line was treated with C2-ceramide. Previous studies have shown that ceramide treatment downregulates myogenic differentiation [61] and MyoG [62], and that the ceramide metabolism was increased in aged sarcoplasmic reticulum [63]. Accordingly, C2C12 myoblasts were treated with 40 µM ceramide in the maintenance condition (DMEM with 10 % FBS and 1 % P/S) and then the medium was changed to a differentiation medium (DMEM with 2% FBS and 1% P/S) to reduce cell proliferation and differentiate myoblasts into myotubes, see Fig. 4A. After 4 days of differentiation, less myotubes were formed and the expression levels of MyoG and MyH3 were decreased by treatment with ceramide, see Fig. 4B-C and Fig. 5A. Especially, the mean brightness of MyH3 staining was significantly decreased by treatment with ceramide (p = 0.035101540), see Fig. 5B.

Transfection of siRNAs was performed to reduce the expression of *ANXA2* and *CLMP. ANXA2* and *CLMP* were identified as upregulated genes in the sarcopenia samples. The transfected *ANXA2* and *CLMP* genes generated more myotubes than the transfected empty siRNA vectors based on the *in vitro* sarcopenia model, see Fig. 4D, which means the downregulation or inhibition of *ANXA2* and *CLMP* expression can be employed in the treatment of sarcopenia by inducing myotubes differentiation.

For the downregulated target genes, the transfected *CYC1* and *SDHB* overexpression plasmid vectors were added into C2C12 cells. The overexpression of *CYC1* and *SDHB* formed more myotubes than transfected empty plasmid vectors based on the *in vitro* sarcopenia model, see Fig. 4E. It was observed that the upregulation of *CYC1* and *SDHB* can be employed in the treatment of sarcopenia by prompting myotubes formation.

### Drug repositioning for target genes

Drug repositioning was performed and identification of effective drug candidates that can modulate the target genes by integrating the available cancer cell lines' transcriptomic signature profiles associated with gene knockdown, knockout, overexpression and chemical perturbation from the CMap database were identified. The drug repositioning approach can be seen in Fig. 6. Drug repositioning analysis was performed for 4 genes, *ANXA2, CLMP, CYC1* and *SDHB.*

In brief, transcriptomic signature profiles associated with the knockdown of *ANXA2* in 8 different cell lines, the knockdown of *CLMP* in 10 different cell lines, the overexpression of *CYC1* in 9 different cell lines and the overexpression of *SDHB* in 10 different cell lines were extracted. The drug-perturbed signature profiles associated with the common 3887, 118, 3887 and 356 drugs for the above cell line sets, respectively, were collected. Based on the Spearman correlation analysis, the similarity matrixes were calculated, where each correlation coefficient represented the similarity of the dysregulation on gene expression induced by the target specific knockdown/knockout/overexpression and drug treatment. After optimization, a simplified similarity matrix for each target gene was obtained, in which each row represented a drug with the best dose and time point, each column represented an optimal shRNA/CRISPR-Cas9 protocol/plasmid in a specific cell line, and each coefficient value indicated the similarity of dysregulation on gene expression induced by a specific drug treatment and a specific target gene knockdown/knockout/overexpression in this cell line. By extracting the drugs ranked in top 1 % based on the similarity coefficients in each cell line, 263 drugs were identified for inhibiting the expression of ANXA2. Then, the coverage for each drug was calculated, which indicates the number of cell lines in which this drug shows a potential inhibition effect. Based on the drug list, the top 10 drugs were extracted based on the highest coverages, see Fig. 7A. This process was repeated by controlling the coefficients within top 5 % and 10 % and coverage within top 10 and then obtained another two drug lists, Figure 7A. Five overlapped drugs were found among the three drug lists, CGP-60474, mitoxantrone, withaferin-A, wortmannin, and dasatinib, and showed an inhibition effect on the expression of *ANXA2* in at least three different cell lines, see Fig. 7A. For example, CGP-60476 showed a potential inhibitory effect in four of the eight cell lines (cell line coverage = 4) and its ranks were within the top 1% based on similarity (correlation coefficients) in each cell line. Similarly, the cell line coverage increased to five and seven, when controlling the correlation coefficient as top 5% and 10%.

Based on the same methodology, the two drugs DL-PDMP and MG-132 showed an inhibitory effect on the expression of *CLMP,* see Fig. 7B. Withaferin-A, mitoxantrone, sirolimus (also known as rapamycin), and midostaurin showed activation on the expression of *Cyc1,* see Fig. 7C, whereas sulforaphane, PIK-75 and vorinostat showed activation on the expression of *SDHB,* see Fig. 7D. Consequently, 12 drugs were selected as the potentially effective drug candidates for the treatment of sarcopenia, see Table 1.

**Table 1 shows the effective drugs for the treatment of sarcopenia predicted by the drug repositioning approach.**

| Target gene | Dysregulation | Function | Treatment | Repurposed drugs |
|---|---|---|---|---|
| *ANXA2* | Up | Annexin A2 | shRNA | Withaferin-a, Wortmannin, Dasatinib, CGP-60474, Mitoxantrone |
| *CLMP* | UP | CXADR like membrane protein | CRISPR-Cas9 | DL-PDMP, MG-132 |
| *CYC1* | Down | Cytochrome c1 | Overexpression | Withaferin-a, Sirolimus (rapamycin), Midostaurin, Mitoxantrone |
| *SDHB* | Down | Succinate dehydrogenase complex iron sulfur subunit B | Overexpression | Sulforaphane, PIK-75, Vorinostat |

### Molecular Docking and Protein-Ligand Interactions

To gain a deeper insight into the binding mode of the repurposed drugs with the selected target protein, docking studies were carried out using the modelled structures of the proteins. The BLAST results presented the best match of templates for the target proteins in terms of identity and query coverage, see Table 2.

**Table 2 shows the protein BLAST results for Sarcopenia targeted genes.**

| Description | Max Score | Total score | Query cover | E value | Identity (%) | PDB Id |
|---|---|---|---|---|---|---|
| *CLMP* | 130 | 130 | 54% | 1e-3 | 32.68 | 3JZ7 |
| *CYC1* | 471 | 471 | 74% | 1e-169 | 100 | 5XTE |
| *SDHB* | 547 | 547 | 100% | 0.0 | 95.71 | 4YTP |

The stereochemical properties of the generated models were evaluated by Ramachandran plot, Table 3.

**Table 3 shows Ramachandran plot statics of the developed models.**

| Models (SWISS model) | Amino acids in most favored regions (%) | Amino acids in additionally allowed regions (%) | Amino acids in generously allowed regions (%) | Amino acids in disallowed Regions (%) |
|---|---|---|---|---|
| *CLMP* | 74.9 | 23.5 | 1.1 | 0.5 |
| *CYC1* | 80.2 | 18.2 | 1.2 | 0.3 |
| *SDHB* | 84.7 | 14.1 | 0.8 | 0.3 |

After evaluation of the models, molecular docking analyses were performed to understand the molecular interaction mechanisms between the target proteins (*ANXA2, CLMP, Cyc1, SDHB*) and drug candidates. Docking analysis indicated that the hit drug candidates had multiple interactions with the crucial residues Asp182, Gln226, and Lys302 of *ANXA2* target. Hydrogen bonds were observed from the polar group (NH, OH, C=O) of drugs while the non-polar group and aromatic rings of the drugs were involved in hydrophobic interactions, see Fig. 8. The molecular interactions for the other target proteins are shown in Fig. 9A-C. Moreover, the binding affinities of the drugs were evaluated as docking scores in Table 4.

**Table 4 shows the docking scores of the drug candidates against the targeted proteins.**

| Protein | Compounds | Docking Score (KCal/mol) |
|---|---|---|
| ANXA2 | Withaferin-A | -7.2546 |
| | Wortmannin | -6.0753 |
| | Dasatinib | -7.6725 |
| | CGP-60474 | -6.8122 |
| | Mitoxantrone | -7.4433 |
| CLMP | DL-PDMP | -6.0826 |
| | MG-132 | -6.5745 |
| CYC1 | Rapamycin | -9.5539 |
| | Withaferin-A | -7.2852 |
| | Midostaurin | -7.8814 |
| | Mitoxantrone | -8.0323 |
| SDHB | Sulforaphane | -4.5989 |
| | PIK-75 | -6.3260 |
| | Vorinostat | -5.9031 |

Drug candidates (withaferin-A, wortmannin, dasatinib, CGP-60474, mitoxantrone) showed moderate binding affinities to the *ANXA2* target protein in the range of 6.08 to 7.67 kcal.mol⁻¹. While rapamycin and mitoxantrone indicated stronger binding affinities (9.55 and 8.03 kcal.mol⁻¹, respectively) to the *CYC1* target protein, the binding affinities of drug candidates for *CLMP* and *SDHB* targets were weaker in the range of 4.60 to 6.58 kcal.mol⁻¹.

### Validation of the drug effect efficacy in C2C12 myoblast cells

To find out the appropriate concentration for treatment, cell viability assays were performed in a dose dependent manner, see Fig.10, and non-toxic doses based on cell viability study were selected.

The C2C12 myoblasts were differentiated to myotubes under treatment of selected concentration of drug candidates. The myoblasts were highly differentiated to myotubes by CGP-60474, DL-PDMP, PIK-75, and withaferin-A, see Fig. 11. The myoblasts were moderately differentiated to myotubes by wortmannin and less differentiated by dasatinib, midostaurin, and mitoxantrone, see Fig. 11.

Further, the CGP-60474, DL-PDMP, PIK-75, withaferin-A and wortmannin were tested on the *in vitro* sarcopenia model based on the C2-ceramide treated C2C12 cell line. Only treatment with withaferin-A improved the myotubes similarly to the control sample (p = 0.70), see Fig. 12 and Fig. 5B. Next, the effect of withaferin-A on the expression of the corresponding target genes was investigated and it was found that the expression level of *ANXA2* was increased by treatment with ceramide, but it was decreased by treatment with withaferin-A, see Fig. 13. Moreover, the myogenic marker MyH3 was expressed as much as control samples with the treatment of withaferin-A, see Fig.13. Thus, it is shown that withaferin-A improves myogenesis by blocking *ANXA2* expression and is a promising drug for the treatment of sarcopenia.

The candidate drugs targeting downregulated genes rarely showed improvements in myotube phenotypic changes. Given the fact that *CYC1* is involved in mitochondrial respiratory chain and is the shared hub genes in mitochondrial process-related modules in three independent cohorts, additional experiments were performed on the *in vitro* sarcopenia model using the compounds carbinoxamine, FR-180204, and ML-167. Non-toxic dosages, see Fig. 14A, were selected to investigate changes in *CYC1* protein levels. Treatment of the *in vitro* sarcopenia model with carbinoxamine, FR-180204, and ML-167 showed promising effect in enhancing myotube cells, see Fig.14B. Treatment with 20 µM carbinoxamine exhibited the large improvement in *CYC1* expression, along with an increase in MyoG and HyH3 expression in the *in vitro* sarcopenia model. FR-180204, a selective extracellular-signal-regulated kinase (ERK) inhibitor, significantly increased the *Cyc1,* MyH3 and MyoG expression at 5 µM. ML-167, a highly selective Cdc2-like kinase inhibitor, led to a notable upregulation of *Cyc1,* MyH3, and MyoG (improved expression) at both tested dosages (2.5 µM and 5 µM). Surprisingly, treatment with FR-180204 and ML-167 also exhibited the ability to facilitate myotube differentiation without the presence of ceramide, see Fig.15.

These experiments show that treatment of sarcopenic cells with withaferin-A, carbinoxamine, FR-180204, or ML-167 show promising results such as upregulation of myogenic markers and are thus very well-suited to be used in the treatment of sarcopenia. FR-180204 and ML-167 further demonstrated the ability to improve the myotube fusion in healthy muscle myoblasts.

### Example 2

This example pertains to drug development based on the interaction with the target genes *PDHX, AGL, SEMA6C, CASQ1, MYORG,* and *CCDC69,* see Fig. 16.

### Methods

### Data and Preparation

The present example utilized data from a previous study on sarcopenia [64]. The cohort has 93 samples, including 19 young healthy, 29 old healthy, 24 old sarcopenic, 4 unclassified bulk RNA-seq and 17 single-nuclei RNA-seq. The 29 old healthy and 24 old sarcopenic samples were chosen for analysis to focus on the differences between sarcopenic and healthy aged populations. In the previous study, Illumina HiSeq 4000 sequencing technology was employed to obtain the used sequence data and the STAR aligner was utilized to generate raw counts, with the GRCh38 reference genome being used [64]. Notably, genes with a total count of less than 10 were excluded from the analysis. Gene count profiles of the cohort were downloaded in CSV format from the Gene Expression Omnibus website (https://www.ncbi.nlm.nih.gov/geo/query/acc.cgi?acc=GSE167186, Access Date: 10 October 2022). These data were subsequently analysed to investigate potential biomarkers of sarcopenia.

### Differential Expression Analysis and Gene Ontology Analysis

DEseq2 package was used in R Studio to perform Differential Expression Analysis [65]. First, a matrix was constructed between metadata and raw count values of sarcopenic and old healthy groups. Then, the analysis was executed with the old healthy group selected as the reference condition to identify differentially expressed genes (DEGs). The Benjamini-Hochberg (BH) approach was used to adjust the false discovery rate (FDR). To find significant DEGs, FDR 0.05 was applied. Gene ontology analysis (GOA) was performed using the "*enrichGo*" function of the *clusterProfiler* package in R, which determines the list of highly enriched genes in biological process pathways by using the hypergeometric distribution [66]. p<0.05 was employed as the cut-off value to find substantially enriched pathways. The *clusterProfiler* sub-package, enrichplot, was operated to visualize GOA results.

### Weighted Gene Co-Expression Network Analysis

The weighted gene co-expression network (GCN) analysis was utilized to establish a GCN using the R package "*WGCNA*" [67]. Metadata and raw count values of the sarcopenia cohort were used as input. Initially, a quality assurance process was conducted by utilizing the "*goodSamplesGenes*" function to eliminate genes with many incomplete records. Then, a clustering tree map was created to identify and eliminate outlier samples. Later, a matrix was created from the input data, and genes which count less than 15 in, along with 75 % of the total samples were removed for downstream analysis. The DEseq2 package was used for variance stabilization and to get normalised counts.

The correlation coefficient was computed between every pair of genes to generate a gene expression similarity matrix, which was then transformed into an adjacency matrix. The "*pickSoftThreshold*" function was utilized to determine the ideal soft thresholding value, resulting in a scale-free network with a scale independence of 0.8. Then, the adjacency matrix was converted to a topological overlap matrix to display the relationships between genes and hierarchical clustering.

Dynamic cut tree methods were employed to identify co-expression gene modules with a minimum connectivity score cut-off of 0.5. These modules that had a positive correlation with the disease state of sarcopenia were termed upregulated modules, and negatively correlated ones were named downregulated modules. In the final step, intramodular analysis was used to identify driver genes by calculating the module membership and the associated p-values.

### Identification of Target Genes

To identify the most influential genes in the upregulated and downregulated modules that were previously identified, a topology analysis was conducted to prioritize hub genes with extensive impacts on the network of modules. All implicated genes' module membership parameters were calculated, and then the topological score was used to arrange all genes into GCN modules in descending order. Afterwards, the top 20 % of genes of each module were extracted.

First, intersections between upregulated-downregulated DEGs and 20 % of upregulated-downregulated modules, respectively, was explored. In the second step, these genes were examined deeper in the Human Protein Atlas Database [68]. Genes that have specific RNA expression levels in muscle tissue were selected as target genes.

### Drug Repositioning

A drug repositioning method, previously developed, based on the similarity analysis of transcriptomics signature profiles of human cells perturbed by drug treatment and genetic treatment [69] was used. The hypothesis of this method is to identify a drug that causes a significant change in gene expression levels in cells that mimics the effect of inhibiting or activating the specific target genes. Based on this method, a T-Drug was constructed (http://drug-reposition.com/), which is a web tool that helps to identify drugs specifically targeting a disease's therapeutic gene [69, 4]. In this web tool, users can query a gene and make predictions about the potentially useful drugs that can inhibit or activate the protein expression of these queried genes.

Drug-perturbation matrix was downloaded for each target gene for the desired effect type in all perturbation data types (shRNA, CRISPR-CAS9, and plasmid overexpression) from the T-drug website with a 0.4 correlation coefficient cut-off. Later, the drugs were sorted in descending order by the number of hit cells criterion, showing how many cell lines where the drug-target association exceeds the correlation coefficient score. Then, the top 200 drugs of each gene were extracted for the following steps. Ultimately, the relationship between each drug's primary target and new targets was inspected using the iNetModels database [70, 71]. Drugs that have a preferred association and can cause upregulation in the expression of the target genes were selected as the drug candidates.

### Cell culture and induction of in vitro sarcopenia model

The C2C12 myoblast line (91031101, Sigma Aldrich, USA) was used for the *in vitro* sarcopenia model. The myoblasts were maintained in modified Eagle's medium (DMEM, D-6429 Sigma Aldrich) supplemented with 10 % FBS (F7524, Sigma-Aldrich), 1 % P/S (P4333, Sigma Aldrich) in a humidified incubator with 5 % CO₂ at 37 °C. C2-ceramide (A7191, Sigma Aldrich) was used for inducing sarcopenia in the C2C12 cell line. After one day of seeding 3×105 cells for each well in a 6-well plate, 40 µM C2-ceramide and the drugs dissolved in 0.1 % DMSO (41639, Sigma-Aldrich) were administered in the maintenance condition for two days. Then, the medium was changed twice, at two-day intervals, with a differentiation medium (DMEM with 2% FBS and 1% P/S) to differentiate myoblasts into myotubes. Because C2-ceramide may affect mitochondrial activity in muscle cells, another cell model was created without introducing ceramide [72]. After completion of myotube differentiation, cells were harvested for western blot analysis.

### Cell Viability Assay

The effects of the drugs on the C2C12 cell viability were tested by MTT (Thiazolyl Blue Tetrazolium Bromide) assay. 1×104 cells were seeded per well in a 96-well plate in 100 µL growth media. After one day of cell seeding, the cells were treated with different concentrations of the drugs dissolved in 0.1 % DMSO and 100 µL of the media for one day. After the treatment period, 5 mg/ml MTT (M2128, Sigma-Aldrich) solution in PBS (10 µL) was added to each well. After one hour, the MTT solution and all media were removed from the wells. 100 µl of DMSO was added and mixed to dissolve the formazan crystals. Cell viability was analysed by measuring the absorbance of the dissolved formazan at a wavelength of 570 nm with a microplate reader (Hidex Sense Beta Plus).

### Western blot analysis

To test the effects of the drugs on the expression of target genes and myogenic markers in the *in vitro* sarcopenia model, the cells were washed with PBS solution and then lysed with CelLytic M (C2978, Sigma-Aldrich) lysis buffer containing protease inhibitors (11836170001, Roche). The cell lysates were centrifuged at 12.000 rpm for 10 min and supernatants were collected. Protein Assay Dye Reagent (5000006, Bio-Rad) was used for the determination of protein concentration. The absorbance of the proteins was measured spectrophotometrically at 595 nm by a microplate reader (Hidex Sense Beta Plus). Protein electrophoresis was performed using Mini-PROTEAN^{®} TGXTM Precast Gels (4561086, Bio-Rad) and then the separated proteins were transferred to a Trans-Blot Turbo Mini 0.2 µm PVDF Transfer Packs membrane (1704158, Bio-Rad) by using Trans-Blot^{®} TurboTM Transfer System (Bio-Rad). The membrane was blocked with 5 % skim milk for 30 min at 4 °C with gentle rocking. After blocking, the membrane was treated with primary antibodies: anti-AGL (ab133720, Abcam, UK) anti-PDHX (HPA038484, Human Protein Atlas, Sweden), anti-Myogenin (ab124800, Abcam), anti-Myosin/MyH3 (ab124205, Abcam), anti-Beta actin (ab8227, Abam) as a loading control overnight at 4 °C on a rocking platform. After the treatment of primary antibodies, the membrane was washed three times with TBS-T buffer (A09-7500-100, Medicago). Then, Goat anti-Rabbit IgG-HRP (ab205718, Abcam) was treated as the secondary antibody for 30 min at 4 °C with gentle rocking. The protein bands on the membrane were revealed using enhanced chemiluminescence substrate (WBLUF0500, Merck) and detected with ImageQuant^{™} LAS 500 (GE Healthcare).

### Results

### Different Patterns of Gene Activity and Specific Functions in Sarcopenia

A gene expression comparison study was performed between sarcopenic and healthy individuals to identify differences using differential expression analysis. Along 36694 genes, 1063 of them have a p< 0.05 and only 26 of them have a p-adjusted < 0.05. 26 DEGs were chosen to be proceeded with. Eight of the DEGs were upregulated and 18 were downregulated in the state of sarcopenia. Furthermore, the functional characteristics of DEGs with increased or decreased expression levels was analysed using gene ontology enrichment analysis. No common biological process pattern was found in 8 upregulated genes. However, downregulated DEGs enriched in muscle contraction, store-operated calcium entry, regulation of cation transmembrane transport, muscle cell development, and other metabolic processes such as glycogen, glucan, and carbohydrate synthesis, see Fig. 17.

### Co-Expression Network Analysis Highlights the Key Modules in Sarcopenia

Co-expression networks were created based on the count data from the cohort study to examine functional gene modules. In the first quality control step, 15279 genes were removed due to too many missing entries. Then, the hierarchical clustering method identified 2 (X_41, X_78) outlier samples and excluded them from the downstream analysis. After normalising the counts with the Deseq2 package, 3 was selected as soft threshold power due to giving high R² results and lower mean connectivity. Next, an adjacency matrix was created, and the topological overlap measure and the dynamic tree cut were employed to form clusters. 18 modules were identified with module sizes ranging between 51 and 1739. All modules consist solely of genes with consensus eigengene connectivity of at least 0.5.

For the following step, the Pearson correlation score between the modules and the samples' disease state of sarcopenia was measured. Out of all the modules tested, 6 exhibited significant outcomes (p<0.05). Along these modules, 2 of them were identified as upregulated (blue and purple); and 4 were classified as downregulated (green, green-yellow, brown, and yellow). Also, gene ontology analysis (GOA) was carried out on the gene modules to determine their related biological pathways. Interestingly, upregulated modules were mainly enriched in mitochondrial energy metabolism. Blue module's GOA results primarily include aerobic respiration, oxidative phosphorylation, and ATP biosynthetic process. Likewise, mitochondrial components were enriched in the purple module such as mitochondrial cytochrome c oxidase assembly, respiratory chain complex IV assembly, and cytochrome complex assembly. Upon examining the downregulated ones, it was observed that they exhibited unique patterns that were different from one another. The green module was enriched in skeletal muscle fibre development, skeletal muscle tissue development and transcription process. Brown module involved with muscle contraction, purine metabolism, mitochondrial respiratory chain subunits and ATP metabolism. The yellow module covered the protein catabolic process and vesicle organization. In despite of including 106 different genes, the green-yellow module did not cluster any specific function in GOA. Lastly, the module membership score was calculated to identify hub nodes using Pearson correlation of the gene expression levels with module eigengenes.

### Determining Target Genes

Examining the structure of a network can reveal the significance of a specific gene within it. Hub genes, which have a high score in topology analysis, are critical components of the network and are believed to be good candidates for further investigation as potential targets. To achieve this, a network topology analysis was conducted by assessing the module membership of genes in both upregulated and downregulated modules. All the genes in each upregulated and downregulated modules were then arranged in descending order based on their module membership parameters and selected the top 20% of genes from each module. As a result, 96 genes from blue and 22 genes from purple were extracted. Subsequently, one gene (*ZFHX3*) was discovered in common in upregulated modules and upregulated DEGs. Likewise, 65 genes were taken from the brown module, 36 genes from the green module, 20 genes from the green-yellow module, and 39 genes from the yellow module. Afterwards, eight genes (*APPL1, PDHX, SEMA6C, AGL, CASQ1, SLC37A4, MYORG,* and *CCDC69*) were found from the intersection between the downregulated modules and downregulated DEGs, see Fig. 18. Furthermore, the genes' tissue RNA expression profiles in The Human Proteome Atlas were investigated. [68] The upregulated target candidate, *ZFHX3,* is mainly enriched in brain tissue. Inside downregulated candidates, *APPL1* shows low tissue specificity and SLC37A4 is mainly enriched in the liver. However, the rest of target candidates, *PDHX, SEMA6C, AGL, CASQ1, MYORG,* and *CCDC69,* are mainly expressed in muscle tissue and are thus the final target genes.

### Drug Repositioning

A drug repositioning approach was used and effective medications that can regulate the target genes were found by combining the sarcopenia transcriptomic signature profiles linked to gene activation, over-expression and chemical alteration from the T-drug database (https://drug-reposition.com/). The repositioning analysis was performed for *PDHX* and AGL because data for 2 out of 6 target genes were solely accessible in the database. The transcriptomic signature profiles related to activating *PDHX* in 13 various cell lines were obtained, and the over-expression of *AGL* in 9 diverse cell lines. Then, gene activation and drug treatment similarity matrix for target genes were downloaded from the database. 6235 drugs and 178 drugs were found to activate *PDHX* and AGL, respectively. Next, the connection between each drug's primary target and our target genes was investigated individually by using the iNetModels website. It was found that clofibric acid (PPAR receptor agonist) targets PPARA which has a positive relationship with *PDHX.* Another drug named Troglitazone (PPAR receptor agonist) has a similar target, PPARG has a positive correlation with *PDHX* as well. One of the withaferin-a (IKK inhibitor) targets, NAMPT shows negative ties *with AGL.* A CDK inhibitor, palbociclib targets CDK4 that has negative correlations with AGL. The target of MG-132 (protease inhibitor), PSMB1 has negative correlation with AGL. Two of Bortezomib's (NFKB pathway inhibitor) targets, PSMB1 and PSMB2 display negative relations with *AGL.* In total, 6 drugs (Palbociclib, MG-132, bortezomib, withaferin-a, clofibric-acid and troglitazone) were found to activate the target genes and were further analysed in *vitro.*

### Validation of the efficacy of drugs in the in vitro sarcopenia model

Before testing the efficacy of the drugs in the *in vitro* sarcopenia model, the effects of different concentrations of each drug on the viability of C2C12 cells were determined and non-toxic optimal doses were selected as follows: palbociclib (5 µM), MG-132 (25 nM), bortezomib (mM), withaferin-a (50 nM), clofibric-acid (5 µM) and troglitazone (0.5 µM), see Fig. 19A. Then, the selected doses of each drug were administered to the *in vitro* sarcopenia model to test the efficacy of the drugs on the expression of the target genes (*AGL* and *PDHX*) and the myogenic markers (MyoG, and MyH3), see Fig.19B.

Furthermore, it was observed that the protein expression levels of *AGL* and *PDHX* in the *in vitro* sarcopenia model were decreased compared to the control. In parallel with the decrease in these target genes, MyoG and MyH3 expression levels were also reduced. Therefore, the downregulated target genes were confirmed in the *in vitro* sarcopenia model.

Furthermore, it was observed that treatment of the *in vitro* sarcopenia model with MG-132 enhanced the expression of *AGL* and treatment with troglitazone enhanced the expression of *PDHX,* see Fig. 20A. Also, it was demonstrated that the myogenic markers were increased compared to the *in vitro* sarcopenia model.

The efficacy of the repurposed drugs was also tested on a cell model that had not been treated with C2-ceramide. All the repurposed drugs, except for Palbociclib, resulted in elevated levels of MyoG and MyH3 expression, see Fig. 20B. That is all the repurposed drugs, except for Palbociclib, exhibited the ability to facilitate myotube differentiation without the presence of ceramide.

These experiments thus show that the expression of the target genes (AGL and *PDHX*) are implicated in sarcopenia and treatment of sarcopenic cells with MG-132 and troglitazone increased the expression of myogenic markers. These compounds are thus very well-suited to be used in the treatment of sarcopenia.

### REFERENCES

[1] von Haehling, S., Morley, J. E. & Anker, S. D. An overview of sarcopenia: facts and numbers on prevalence and clinical impact. J Cachexia Sarcopenia Muscle 1, 129-133, doi:10.1007/s13539-010-0014-2 (2010).
[2] Curcio, F. et al. Biomarkers in sarcopenia: A multifactorial approach. Exp Gerontol 85, 1-8, doi:10.1016/j.exger.2016.09.007 (2016).
[3] van Dam, S., Vosa, U., van der Graaf, A., Franke, L. & de Magalhaes, J. P. Gene co-expression analysis for functional classification and gene-disease predictions. Brief Bioinform 19, 575-592, doi:10.1093/bib/bbw139 (2018).
[4] Lee, S. et al. Integrated Network Analysis Reveals an Association between Plasma Mannose Levels and Insulin Resistance. Cell Metabolism 24, 172-184, doi:https://doi.org/10.1016/j.cmet.2016.05,026 (2016).
[5] Arif, M. et al. Integrative transcriptomic analysis of tissue-specific metabolic crosstalk after myocardial infarction. Elife 10, doi:10.7554/eLife.66921 (2021).
[6] Altay, O., Nielsen, J., Uhlen, M., Boren, J. & Mardinoglu, A. Systems biology perspective for studying the gut microbiota in human physiology and liver diseases. EBioMedicine 49, 364-373, doi:10.1016/j.ebiom.2019.09.057 (2019).
[7] Altay, O. et al. Revealing the Metabolic Alterations during Biofilm Development of Burkholderia cenocepacia Based on Genome-Scale Metabolic Modeling. Metabolites 11, doi:10.3390/metabo11040221 (2021).
[8] Kwak, J. Y. & Kwon, K.-S. Pharmacological Interventions for Treatment of Sarcopenia: Current Status of Drug Development for Sarcopenia. Ann Geriatr Med Res 23, 98-104, doi:10.4235/agmr.19.0028 (2019).
[9] C. Zhang et al., Discovery of therapeutic agents targeting PKLR for NAFLD using drug repositioning. EBioMedicine 83, 104214 (2022).
[10] S. Doran et al., Multi-omics approaches for revealing the complexity of cardiovascular disease. Brief Bioinform 22, (2021).
[11] J. Sung, Y. Wang, S. Chandrasekaran, D. M. Witten, N. D. Price, Molecular signatures from omics data: from chaos to consensus. Biotechnol J 7, 946-957 (2012).
[12] X. Li et al., Stratification of patients with clear cell renal cell carcinoma to facilitate drug repositioning. iScience 24, 102722 (2021).
[13] Arena, I. G., Pugliese, A., Volta, S., Toscano, A. & Musumeci, O. Molecular Genetics Overview of Primary Mitochondrial Myopathies. J Clin Med 11, doi:10.3390/jcm11030632 (2022).
[14] Nolfi-Donegan, D., Braganza, A. & Shiva, S. Mitochondrial electron transport chain: Oxidative phosphorylation, oxidant production, and methods of measurement. Redox Biol 37, 101674, doi:10.1016/j.redox.2020.101674 (2020).
[15] Kang, Y.-J., Yoo, J.-I. & Baek, K.-W. Differential gene expression profile by RNA sequencing study of elderly osteoporotic hip fracture patients with sarcopenia. Journal of Orthopaedic Translation 29, 10-18, doi:https://doi.org/10.1016/j.jot.2021.04.009 (2021).
[16] Enrich, C., Lu, A., Tebar, F., Rentero, C. & Grewal, T. Annexins Bridging the Gap: Novel Roles in Membrane Contact Site Formation. Frontiers in Cell and Developmental Biology 9, doi:10.3389/fcell.2021.797949 (2022).
[17] Croissant, C., Carmeille, R., Brévart, C. & Bouter, A. Annexins and Membrane Repair Dysfunctions in Muscular Dystrophies. International Journal of Molecular Sciences 22, 5276 (2021).
[18] Cagliani, R. et al. Mutation finding in patients with dysferlin deficiency and role of the dysferlin interacting proteins annexin A1 and A2 in muscular dystrophies. Hum Mutat 26, 283, doi:10.1002/humu.9364 (2005).
[19] Hogarth, M. W. et al. Fibroadipogenic progenitors are responsible for muscle loss in limb girdle muscular dystrophy 2B. Nat Commun 10, 2430, doi:10.1038/s41467-019-10438-z (2019).
[20] Foltz, S. J., Cui, Y. Y., Choo, H. J. & Hartzell, H. C. ANO5 ensures trafficking of annexins in wounded myofibers. J Cell Biol 220, doi:10.1083/jcb.202007059 (2021).
[21] Langer, H. T. et al. Generation of desminopathy in rats using CRISPR-Cas9. J Cachexia Sarcopenia Muscle 11, 1364-1376, doi:https://doi.org/10.1002/jcsm.12619 (2020).
[22] J. Inoue et al., Identification of PDHX as a metabolic target for esophageal squamous cell carcinoma. Cancer Sci 112, 2792-2802 (2021).
[23] L. L. Spriet, G. J. Heigenhauser, Regulation of pyruvate dehydrogenase (PDH) activity in human skeletal muscle during exercise. Exerc Sport Sci Rev 30, 91-95 (2002).
[24] P. M. Coen, R. V. Musci, J. M. Hinkley, B. F. Miller, Mitochondria as a Target for Mitigating Sarcopenia. Frontiers in Physiology 9, (2019).
[25] M. Miné et al., A novel gross deletion caused by non-homologous recombination of the PDHX gene in a patient with pyruvate dehydrogenase deficiency. Mol Genet Metab 89, 106-110 (2006).
[26] Y. Endo et al., Molecular analysis of the AGL gene: heterogeneity of mutations in patients with glycogen storage disease type III from Germany, Canada, Afghanistan, Iran, and Turkey. Journal of Human Genetics 51, 958-963 (2006).
[27] J. L. Goldstein et al., Molecular analysis of the AGL gene: identification of 25 novel mutations and evidence of genetic heterogeneity in patients with Glycogen Storage Disease Type III. Genet Med 12, 424-430 (2010).
[28] J. Shen et al., Mutations in exon 3 of the glycogen debranching enzyme gene are associated with glycogen storage disease type III that is differentially expressed in liver and muscle. J Clin Invest 98, 352-357 (1996).
[29] P. S. Kishnani et al., Glycogen storage disease type III diagnosis and management guidelines. Genet Med 12, 446-463 (2010).
[30] Lee, I.-C. & Choi, B. Y. Withaferin-A--A Natural Anticancer Agent with Pleitropic Mechanisms of Action. International journal of molecular sciences 17, 290-290, doi:10.3390/ijms17030290 (2016).
[31] Ozorowski, G., Ryan, C. M., Whitelegge, J. P. & Luecke, H. Withaferin A binds covalently to the N-terminal domain of annexin A2. Biol Chem 393, 1151-1163, doi:10.1515/hsz-2012-0184 (2012).
[32] Dutta, R., Khalil, R., Green, R., Mohapatra, S. S. & Mohapatra, S. Withania Somnifera (Ashwagandha) and Withaferin A: Potential in Integrative Oncology. Int J Mol Sci 20, doi:10.3390/ijms20215310 (2019).
[33] Theret, M. et al. In vitro assessment of anti-fibrotic drug activity does not predict in vivo efficacy in murine models of Duchenne muscular dystrophy. Life Sciences 279, 119482, doi:https://doi.org/10.1016/j.lfs.2021.119482 (2021).
[34] Simons, F. E. & Simons, K. J. H1 antihistamines: current status and future directions. World Allergy Organ J 1, 145-155, doi:10.1186/1939-4551-1-9-145 (2008).
[35] Ohori, M., Takeuchi, M., Maruki, R., Nakajima, H. & Miyake, H. FR180204, a novel and selective inhibitor of extracellular signal-regulated kinase, ameliorates collagen-induced arthritis in mice. Naunyn Schmiedebergs Arch Pharmacol 374, 311-316, doi:10.1007/s00210-006-0117-7 (2007).
[36] Rosenthal, A. S. et al. in Probe Reports from the NIH Molecular Libraries Program (National Center for Biotechnology Information (US), 2010).
[37] D. H. Lee, A. L. Goldberg, Proteasome inhibitors: valuable new tools for cell biologists. Trends Cell Biol 8, 397-403 (1998).
[38] K. Sakuma, A. Yamaguchi, Novel intriguing strategies attenuating to sarcopenia. J Aging Res 2012, 251217 (2012).
[39] H. Sakai et al., Upregulation of ubiquitinated proteins and their degradation pathway in muscle atrophy induced by cisplatin in mice. Toxicol Appl Pharmacol 403, 115165 (2020).
[40] C. M. Spencer, A. Markham, Troglitazone. Drugs 54, 89-101 (1997).
[41] R. H. Unger, L. Orci, Lipoapoptosis: its mechanism and its diseases. Biochim Biophys Acta 1585, 202-212 (2002).
[42] Migliavacca, E. et al. Mitochondrial oxidative capacity and NAD+ biosynthesis are reduced in human sarcopenia across ethnicities. Nature Communications 10, 5808, doi:10.1038/s41467-019-13694-1 (2019).
[43] Virtanen, P. et al. SciPy 1.0: fundamental algorithms for scientific computing in Python. Nature methods 17, 261-272 (2020).
[44] Shannon, P. et al. Cytoscape: a software environment for integrated models of biomolecular interaction networks. Genome Res 13, 2498-2504, doi:10.1101/gr.1239303 (2003).
[45] Li, X. et al. Prediction of drug candidates for clear cell renal cell carcinoma using a systems biology-based drug repositioning approach. EBioMedicine 78, 103963, doi:10.1016/j.ebiom.2022.103963 (2022).
[46] Shao, C. et al. Crystallographic analysis of calcium-dependent heparin binding to annexin A2. J Biol Chem 281, 31689-31695, doi:10.1074/jbc.M604502200 (2006).
[47] Waterhouse, A. et al. SWISS-MODEL: homology modelling of protein structures and complexes. Nucleic Acids Res 46, W296-W303, doi:10.1093/nar/gky427 (2018).
[48] Laskowski, R. A., Rullmannn, J. A., MacArthur, M. W., Kaptein, R. & Thornton, J. M. AQUA and PROCHECK-NMR: programs for checking the quality of protein structures solved by NMR. J Biomol NMR 8, 477-486, doi:10.1007/bf00228148 (1996).
[49] Eisenberg, D., Lüthy, R. & Bowie, J. U. VERIFY3D: assessment of protein models with three-dimensional profiles. Methods Enzymol 277, 396-404, doi:10.1016/s0076-6879(97)77022-8 (1997).
[50] Colovos, C. & Yeates, T. O. Verification of protein structures: patterns of nonbonded atomic interactions. Protein Sci 2, 1511-1519, doi:10.1002/pro.5560020916 (1993).
[51] Halgren, T. A. Merck molecular force field. I. Basis, form, scope, parameterization, and performance of MMFF94. Journal of Computational Chemistry 17, 490-519, doi:https://doi.org/10.1002/(SICI)1096-987X(199604)17:5/6<490::AID-JCC1>3.0.CO;2-P (1996).
[52] Dillon, E. L. in The Molecular Nutrition of Amino Acids and Proteins (ed Dominique Dardevet) 79-97 (Academic Press, 2016).
[53] González-Blanco, L. et al. Cell interactome in sarcopenia during aging. Journal of Cachexia, Sarcopenia and Muscle 13, 919-931, doi:https://doi.org/10.1002/icsm.12937 (2022).
[54] Bua, E. A., McKiernan, S. H., Wanagat, J., McKenzie, D. & Aiken, J. M. Mitochondrial abnormalities are more frequent in muscles undergoing sarcopenia. Journal of Applied Physiology 92, 2617-2624, doi:10.1152/japplphysiol.01102.2001 (2002).
[55] Joseph, A.-M. et al. The impact of aging on mitochondrial function and biogenesis pathways in skeletal muscle of sedentary high- and low-functioning elderly individuals. Aging Cell 11, 801-809, doi:https://doi.org/10.1111/j.1474-9726.2012.00844.x (2012).
[56] Chabi, B. et al. Mitochondrial function and apoptotic susceptibility in aging skeletal muscle. Aging Cell 7, 2-12, doi:https://doi.org/10.1111/j.1474-9726.2007.00347.x (2008).
[57] Grindheim, A. K., Saraste, J. & Vedeler, A. Protein phosphorylation and its role in the regulation of Annexin A2 function. Biochimica et Biophysica Acta (BBA) - General Subjects 1861, 2515-2529, doi:https://doi.org/10.1016/j.bbagen.2017.08.024 (2017).
[58] Han, X. et al. CXADR-like membrane protein protects against heart injury by preventing excessive pyroptosis after myocardial infarction. J Cell Mol Med 24, 13775-13788, doi:10.1111/jcmm.15955 (2020).
[59] Gaignard, P. et al. Mutations in CYC1, encoding cytochrome c1 subunit of respiratory chain complex III, cause insulin-responsive hyperglycemia. American journal of human genetics 93, 384-389, doi:10.1016/j.ajhg.2013.06.015 (2013).
[60] Punsoni, M. et al. Succinate Dehydrogenase B (SDHB) Immunohistochemistry for the Evaluation of Muscle Biopsies. Appl Immunohistochem Mol Morphol 25, 645-650, doi:10.1097/PAI.0000000000000432 (2017).
[61] Meadows, K. A., Holly, J. M. & Stewart, C. E. Tumor necrosis factor-alpha-induced apoptosis is associated with suppression of insulin-like growth factor binding protein-5 secretion in differentiating murine skeletal myoblasts. J Cell Physiol 183, 330-337, doi:10.1002/(SICI)1097-4652(200006)183:3<330::AID-JCP5>3.0.CO;2-N (2000).
[62] Mebarek, S. et al. Inhibition of de novo ceramide synthesis upregulates phospholipase D and enhances myogenic differentiation. J Cell Sci 120, 407-416, doi:10.1242/jcs.03331 (2007).
[63] Russ, D. W., Wills, A. M., Boyd, I. M. & Krause, J. Weakness, SR function and stress in gastrocnemius muscles of aged male rats. Exp Gerontol 50, 40-44, doi:10.1016/j.exger.2013.11.018 (2014).
[64] K. Perez et al., Single nuclei profiling identifies cell specific markers of skeletal muscle aging, frailty, and senescence. Aging (Albany NY) 14, 9393-9422 (2022).
[65] M. I. Love, W. Huber, S. Anders, Moderated estimation of fold change and dispersion for RNA-seq data with DESeq2. Genome Biology 15, 550 (2014).
[66] G. Yu, L. G. Wang, Y. Han, Q. Y. He, clusterProfiler: an R package for comparing biological themes among gene clusters. Omics 16, 284-287 (2012).
[67] P. Langfelder, S. Horvath, WGCNA: an R package for weighted correlation network analysis. BMC Bioinformatics 9, 559 (2008).
[68] M. Uhlén et al., Proteomics. Tissue-based map of the human proteome. Science 347, 1260419 (2015).
[69] X. Li et al., Prediction of drug candidates for clear cell renal cell carcinoma using a systems biology-based drug repositioning approach. EBioMedicine 78, 103963 (2022).
[70] M. Arif et al., iNetModels 2.0: an interactive visualization and database of multi-omics data. Nucleic Acids Res 49, W271-w276 (2021).
[71] S. Lee et al., TCSBN: a database of tissue and cancer specific biological networks. Nucleic Acids Res 46, D595-d600 (2018).
[72] T. I. Lima et al., Inhibiting de novo ceramide synthesis restores mitochondrial and protein homeostasis in muscle aging. Sci Transl Med 15, eade6509 (2023).

## Claims

1. A compound or a pharmaceutically acceptable salt or prodrug thereof for use in a method of treatment of sarcopenia wherein the compound is selected from carbinoxamine, FR-180204, ML-167, withaferin-A, MG-132 and troglitazone.

2. The compound according to claim 1, wherein the compound is withaferin-A.

3. The compound according to claim 2, wherein the method of treatment further comprises administration of an NF-κB inhibitor.

4. The compound according to claim 1, wherein the compound is carbinoxamine.

5. The compound according to claim 1, wherein the compound is FR-180204.

6. The compound according to claim 1, wherein the compound is ML-167.

7. The compound according to claim 1, wherein the compound is MG-132.

8. The compound according to claim 1, wherein the compound is troglitazone.
